# EUROPEAN PATENT SPECIFICATION

(11) **EP 2 632 424 B1**
(45) Date of publication and mention of the grant of the patent: **23.08.2017**
(21) Application number: 11773003.6
(22) Date of filing: 24.10.2011
(51) Int. Cl.: A61K 8/58, A61K 8/41, A61K 8/60, A61Q 5/02, A61Q 5/12

(54) **COSMETIC COMPOSITION COMPRISING A FATTY-CHAIN ALKOXYSILANE AND A CATIONIC OR NONIONIC SURFACTANT.**
KOSMETISCHE ZUSAMMENSETZUNG MIT EINEM FETTSÄUREKETTEN-ALKOXYSILAN UND EINEM KATIONISCHEN ODER NICHTIONISCHEN TENSID
COMPOSITION COSMÉTIQUE COMPRENANT UN ALCOXYSILANE À CHAÎNE GRASSE ET UN TENSIOACTIF CATIONIQUE OU NON IONIQUE

(30) Priority: 08.11.2010 US 411075 P; 08.11.2010 US 411064 P; 26.10.2010 FR 1058803; 26.10.2010 FR 1058799
(43) Date of publication of application: 04.09.2013
(73) Proprietor: L'Oréal, 75008 Paris (FR)
(72) Inventor: KHENNICHE, Samira, F-92110 Clichy (FR); PLOS, Grégory, F-75018 Paris (FR); ROLLAT-CORVOL, Isabelle, F-75017 Paris (FR)
(74) Representative: Casalonga
(86) International application number: PCT/EP2011/068530
(87) International publication number: WO 2012/055811

(56) References cited:
- EP-A1- 1 736 139
- EP-A2- 0 159 628
- WO-A1-2004/012691
- DATABASE gnpd [Online] Mintel; November 2010 (2010-11), "3-Minute Shine Boost", XP002642524, Database accession no. 1442607
- Anonymous: "Alterna Caviar Anti-Aging 3-Minute Shine Boost", , 18 October 2010 (2010-10-18), XP002642525, Retrieved from the Internet: URL:http://www.folica.com/hair-care/hair-t reatments [retrieved on 2011-06-20]
- DATABASE gnpd [Online] Mintel; September 2009 (2009-09), "Conditioner with Caviar Enriched Proteins", XP002642526, Database accession no. 1183212
- DATABASE gnpd [Online] Mintel; September 2010 (2010-09), "Shine Conditioner", XP002644015, Database accession no. 1397473
- DATABASE gnpd [Online] Mintel; September 2010 (2010-09), "Strengthening and Nourishing Conditioner", XP002644016, Database accession no. 1464288
- DATABASE gnpd [Online] Mintel; March 2010 (2010-03), "Conditioner", XP002639303, Database accession no. 1300395
- "Conditioner", GNPD,, 1 September 2010 (2010-09-01), XP002639305,
- "Rich & Delicious Intense Caviar Hair Masque", GNPD,, 1 September 2009 (2009-09-01), XP002639302,

## Description

The present invention relates to a cosmetic composition comprising, in a cosmetically acceptable medium, one or more fatty-chain alkoxysilanes, one or more surfactants chosen from cationic surfactants and nonionic surfactants, and one or more alkoxysilanes other the above mentioned fatty-chain alkoxysilanes.

The present invention also relates to the use of the said composition for caring for keratin materials, such as human skin and human keratin fibres and in particular the hair, and also to a cosmetic process for treating keratin materials using such a composition.

A recurrent problem in the field of cosmetic haircare consists in caring for keratin fibres subjected to various external attacking factors. Specifically, these fibres may be subject to attack of various origins, such as mechanical attack, for example linked to disentangling or blow-drying, or alternatively chemical attack, for example following dyeing or permanent-waving.

Attack due to chemical and mechanical treatments has consequences especially on the qualities of the keratin fibre and may lead to difficult disentangling at the time of washing the hair, on dry hair and/or wet hair, and also to degradation of the surface properties of the fibres, which become non-smooth and irregular at the surface, more particularly when the hair is dry.

Care products exist that can limit these phenomena. However, these solutions are not always sufficiently effective and are not sufficiently shampoo-resistant.

The prior-art compositions that can facilitate disentangling by softening the keratin fibre and that afford gloss, softness and uniformity to dry hair essentially comprise cationic surfactants, fatty substances, silicones and cationic polymers.

After having been applied, these compositions are rinsed out, and the cosmetic agents, which are only lightly deposited on the keratin fibres, are generally removed at the time of the next wash.

Thus, the application of these compositions must be repeated after each wash, in order to treat the hair and to facilitate its conditioning.

There is thus a need for compositions that can form a material or that can deposit care active agents uniformly onto and/or into the keratin fibres, in a manner that is resistant to shampooing several times.

Furthermore, the consumers wish to be able to wash their faces and body without drying their skin, and potentially treating it in order to give a good slip to their skin.

Products exist, that are called shower gels, based on anionic surfactants. However such compositions often leave an insufficient deposit on the skin. It is possible to improve the treating effect of such compositions by adding cationic compounds. However, this solution is not always fully satisfying considering the eco-toxicity of the materials used.

Hair conditioning products have been marketed under the names "Rich & Delicious Intense Caviar Hair Masque'', "Conditioner with Caviar Enriched Proteins" and "3-minute Shine Boost", that contain among other ingredients surfactants and triethoxycaprylylsilane.

Furthermore, different shampoo and hair conditioning compositions have been marketed containing mixtures of different types of surfactants including in particular cationic surfactants.

International patent application WO 2004/012 691 discloses the cosmetic use of silanes for improving the condition of the hair.

Patent application EP 0 159 628 proposes compositions for reinforcing the elasticity of the hair, comprising an alkyltrialkoxysilane.

Moreover, patent application EP 1 736 139 describes a hair treatment composition comprising an alkoxysilane, an organic acid and water, the pH of the composition being between 2 and 5.

Finally, patent application EP 0 877 027 discloses a composition comprising an organosilane and a particular polyol.

The Applicant has now discovered, surprisingly, that a combination of a particular fatty-chain alkoxysilane with at least one cationic and/or nonionic surfactant can afford efficient and long-lasting treatment of the hair and the skin, and thus facilitate their conditioning.

In particular, such a composition can, firstly, treat the hair, and, secondly, give cosmetic effects that are resistant to shampooing several times.

One subject of the present invention is thus a cosmetic composition comprising:
- at least 0.1% by weight, relative to the total weight of the composition, of one or more fatty-chain alkoxysilanes of formula (I) below:

   R₁Si(OR₂)₃ (I)

   in which R₁ represents a linear or branched alkyl or alkenyl group comprising from 7 to 18 carbon atoms, and R₂ represents a linear or branched alkyl group comprising from 1 to 6 carbon atoms,
- one or more surfactant(s) chosen from cationic surfactants and nonionic surfactants, and mixtures thereof, and
- one or more alkoxysilane(s), other than the alkoxysilanes of formula (I), chosen from compounds of formula (VII) below: 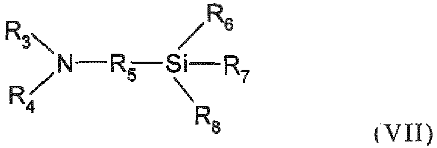 in which:
   R₆ represents a halogen or a group OR' or R'₆,
   R₇ represents a halogen or a group OR" or R'₇,
   R₈ represents a halogen or a group OR'" or R'₈,
   R₃, R₄, R₅, R', R", R''', R'₆, R'₇ and R'₈ represent, independently of each other, a saturated or unsaturated, linear or branched hydrocarbon-based group, optionally bearing additional chemical groups, R₃, R₄, R', R" and R''' also possibly denoting hydrogen, at least two of the groups R₆, R₇ and R₈ being different from the groups R'₆, R'₇ and R'₈, at least two of the groups R', R" and R''' being other than hydrogen.

The present invention further concerns a cosmetic composition, obtainable by mixing the following ingredients:
- at least 0.1% by weight, relative to the total weight of the composition, of one or more fatty-chain alkoxysilanes of formula (I) below:

   R₁Si(OR₂)₃ (I)

   in which R₁ represents a linear or branched alkyl or alkenyl group comprising from 7 to 18 carbon atoms, and R₂ represents a linear or branched alkyl group comprising from 1 to 6 carbon atoms,
- one or more surfactant(s) chosen from cationic surfactants and nonionic surfactants, and mixtures thereof, and
- one or more alkoxysilane(s), other than the alkoxysilanes of formula (I), chosen from compounds of formula (VII) below: 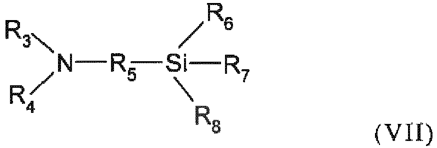 in which:
   R₆ represents a halogen or a group OR' or R'₆,
   R₇ represents a halogen or a group OR" or R'₇,
   R₈ represents a halogen or a group OR'" or R'₈,
   R₃, R₄, R₅, R', R", R''', R'₆, R'₇ and R'₈ represent, independently of each other, a saturated or unsaturated, linear or branched hydrocarbon-based group, optionally bearing additional chemical groups, R₃, R₄, R', R" and R''' also possibly denoting hydrogen, at least two of the groups R₆, R₇, and R₈ being different from the groups R'₆, R'₇ and R'₈, at least two of the groups R', R" and R''' being other than hydrogen.

The composition of the present invention proves to be particularly suitable for caring for the hair and the skin and also makes it possible to obtain very good working qualities such as particularly easy application and good rinseability.

The composition according to the invention gives the hair excellent cosmetic properties, and in particular promotes the disentangling, suppleness and smoothness of the hair.

In addition, the feel of the hair and of the skin after treatment using the composition according to the invention is particularly pleasant.

Finally, the conditioning properties obtained using the composition according to the invention are shampoo- and washing-resistant.

Another subject of the invention consists of a cosmetic process for treating keratin materials, especially human skin and human keratin fibres such as the hair, which consists in applying to the hair an effective amount of a composition according to the invention.

In particular, the composition according to the invention may be rinsed out or left in, optionally applied under the effect of heat, and optionally combined with chemical and/or mechanical hair treatments.

A subject of the invention is also the use of the composition according to the invention for caring for keratin materials, such as human skin and human keratin fibres such as in particular the hair.

Other subject-matters, characteristics, aspects and advantages of the invention will become even more clearly apparent on reading the description which follows.

According to the present invention, the composition comprises one or more fatty-chain alkoxysilanes.

The fatty-chain alkoxysilane(s) that may be used in the composition according to the invention are those corresponding to formula (I) below:

R₁Si(OR₂)₃ (I)

in which R¹ represents a linear or branched alkyl or alkenyl group comprising from 7 to 18 carbon atoms, and R₂ represents a linear or branched alkyl group comprising from 1 to 6 carbon atoms and preferably from 1 to 4 carbon atoms, and even more preferentially the ethyl group.

Said fatty-chain alkoxysilane can be present in the composition under the form of a compound of formula (I) above, and/or under the form of one or more oligomer(s) of such a compound.

In formula (I) above, R₁ being an alkyl or alkenyl group and R₂ being an alkyl group, these groups comprise only carbon and hydrogen atoms.

Preferably, R₂ represents an alkyl group comprising from 1 to 4 carbon atoms, better still a linear alkyl group comprising from 1 to 4 carbon atoms, and preferably the ethyl group.

Preferably, R₁ represents an alkyl group and even more preferentially a linear alkyl group.

Preferably, the fatty-chain alkoxysilane is chosen from octyltriethoxysilane, dodecyltriethoxysilane, octadecyltriethoxysilane and hexadecyltriethoxysilane.

More particularly, the fatty-chain alkoxysilane according to the invention is octyltriethoxysilane (OTES).

The alkoxysilane(s) of formula (I) are present in the composition according to the invention in proportions of at least 0.1% by weight, preferably ranging from 0.1 to 20% by weight, more preferably from 0.5 to 18% by weight and better still from 2 to 15% by weight, relative to the total weight of the composition.

According to a first embodiment of the invention, the composition comprises one or more cationic surfactants.

The cationic surfactant(s) that can be used in the compositions of the present invention comprise, for example, salts of optionally polyoxyalkylenated primary, secondary or tertiary fatty amines, quaternary ammonium salts, and mixtures thereof.

Examples of quaternary ammonium salts that may especially be mentioned include:
- those corresponding to the general formula (II) below: 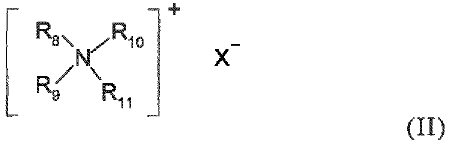 in which the groups R₈ to R₁₁, which may be identical or different, represent a linear or branched aliphatic group comprising from 1 to 30 carbon atoms or an aromatic group such as aryl or alkylaryl, at least one of the groups R₈ to R₁₁ comprising from 8 to 30 carbon atoms and preferably from 12 to 24 carbon atoms. The aliphatic groups may comprise heteroatoms such as, in particular, oxygen, nitrogen, sulfur and halogens.

The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkylacetate, C₁-C₃₀ hydroxyalkyl, X- is an anionic counterion chosen from halides, phosphates, acetates, lactates, (C₁-C₄)alkyl sulfates, and (C₁-C₄)alkyl-or (C₁-C₄)alkylarylsulfonates.

Preference is given, among the quaternary ammonium salts of formula (II), on the one hand, to tetraalkylammonium chlorides, such as, for example, dialkyldimethylammonium or alkyltrimethylammonium chlorides in which the alkyl group comprises approximately from 12 to 22 carbon atoms, in particular behenyltrimethylammonium chloride, distearyldimethylammonium chloride, cetyltrimethylammonium chloride or benzyldimethylstearyl-ammonium chloride, or also, on the other hand, to distearoylethylhydroxyethylmethylammonium methosulfate, dipalmitoylethylhydroxyethylarnmonium methosulfate or distearoylethylhydroxyethylammonium methosulfate, or also, finally, to palmitylamidopropyltrimethylammonium chloride or stearamidopropyldimethyl(myristyl acetate)ammonium chloride, sold under the name Ceraphyl^{®} 70 by Van Dyk.
- quaternary ammonium salts of imidazoline, for instance those of formula (III) below: 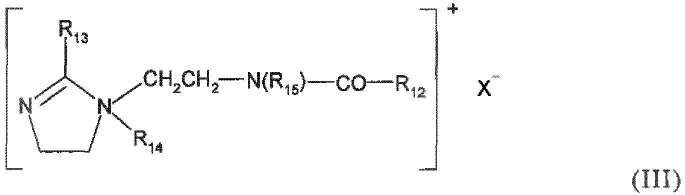 in which R₁₂ represents an alkyl or alkenyl group comprising from 8 to 30 carbon atoms, for example fatty acid derivatives of tallow, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X⁻ is an anion chosen from the group of halides, phosphates, acetates, lactates, (C₁-C₄)alkyl sulfates and (C₁-C₄)alkyl-or (C₁-C₄)alkylaryl-sulfonates. R₁₂ and R₁₃ preferably denote a mixture of alkyl or alkenyl groups comprising from 12 to 21 carbon atoms, for example tallow fatty acid derivatives, R₁₄ denotes a methyl group, and R₁₅ denotes a hydrogen atom. Such a product is, for example, sold under the name Rewoquat® W 75 by the company Rewo,
- quaternary diammonium or triammonium salts, particularly of formula (IV) below: 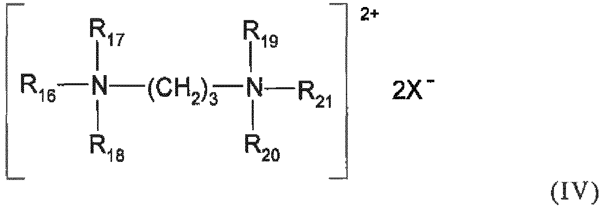 in which R₁₆ denotes an alkyl group comprising from about 16 to 30 carbon atoms, optionally hydroxylated and/or interrupted with one or more oxygen atoms; R₁₇ is chosen from hydrogen, an alkyl group comprising from 1 to 4 carbon atoms or a group -(CH₂)₃-N⁺(R₁₆ₐ)(R₁₇ₐ)(R₁₈ₐ); R₁₆ₐ, R₁₇ₐ, R₁₈ₐ, R₁₈, R₁₉, R₂₀ and R₂₁, which may be identical or different, are chosen from hydrogen and an alkyl group comprising from 1 to 4 carbon atoms, and X⁻ is an anion chosen from the group of halides, acetates, phosphates, nitrates, (C₁-C₄)alkyl sulfates and (C₁-C₄)alkyl- or (C₁-C₄)alkylaryl-sulfonates, in particular methyl sulfate and ethyl sulfate. Such compounds are, for example, Finquat CT-P, available from the company Finetex (Quaternium 89), and Finquat CT, available from the company Finetex (Quaternium 75),
- quaternary ammonium salts comprising one or more ester functions, for instance those of formula (V) below: 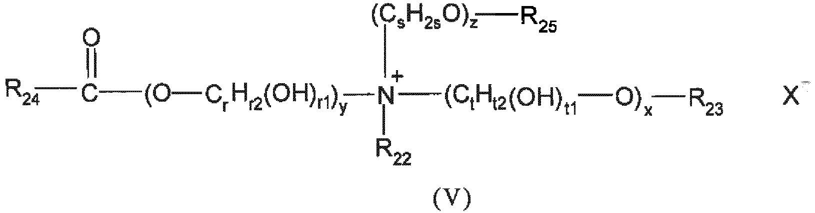 in which:
   R₂₂ is chosen from C₁-C₆ alkyl and C₁-C₆ hydroxyalkyl or dihydroxyalkyl groups;
   R₂₃ is chosen from:
      - the group 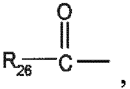
      - linear or branched, saturated or unsaturated C₁-C₂₂ hydrocarbon-based groups R₂₇,
      - a hydrogen atom,
   R₂₅ is chosen from:
      - the group 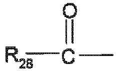
      - linear or branched, saturated or unsaturated C₁-C₆ hydrocarbon-based groups R₂₉,
      - a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6,
   r1 and t1, which may be identical or different, are equal to 0 or 1,
   r2 + r1 = 2r and t1 + t2 = 2t,
   y is an integer ranging from 1 to 10,
   x and z, which may be identical or different, are integers ranging from 0 to 10,
   X⁻ is a simple or complex, organic or mineral anion,
   with the proviso that the sum x + y + z is from 1 to 15, that when x is 0, then R₂₃ denotes R₂₇ and that when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is a hydrocarbon-based group R₂₇, it may be long and contain from 12 to 22 carbon atoms, or may be short and contain from 1 to 3 carbon atoms.

When R₂₅ is a hydrocarbon-based group R₂₉, it preferably contains 1 to 3 carbon atoms.

Advantageously, R₂₄, R₂₆ and R₂₈, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X⁻ is preferably a halide, preferably chloride, bromide or iodide, a (C₁-C₄)alkyl sulfate or a (C₁-C₄)alkyl- or (C₁-C₄)alkylaryl-sulfonate. However, methanesulfonate, phosphate, nitrate, tosylate, an anion derived from an organic acid, such as acetate or lactate, or any other anion that is compatible with the ammonium containing an ester function, may be used.

The anion X⁻ is even more particularly chloride, methyl sulfate or ethyl sulfate.

Use is made more particularly in the composition according to the invention of the ammonium salts of formula (V) in which:
- R₂₂ denotes a methyl or ethyl group,
- x and y are equal to 1,
- z is equal to 0 or 1,
- r, s and t are equal to 2,
- R₂₃ is chosen from:
- the group 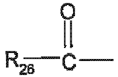
- methyl, ethyl or C₁₄-C₂₂ hydrocarbon-based groups,
- a hydrogen atom,
- R₂₅ is chosen from:
- the group 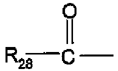
- a hydrogen atom,
- R₂₄, R₂₆ and R₂₈, which may be identical or different, are chosen from linear or branched, saturated or unsaturated C₁₃-C₁₇ hydrocarbon-based groups, and more particularly from linear or branched, saturated or unsaturated C₁₃-C₁₇ alkyl and alkenyl groups.

The hydrocarbon-based groups are advantageously linear.

Among the compounds of formula (V), examples that may be mentioned include salts, especially the chloride or methyl sulfate, of diacyloxyethyldimethylammonium, diacyloxyethylhydroxyethylmethylammonium, monoacyloxyethyldihydroxyethylmethyl - ammonium, triacyloxyethylmethylammonium or monoacyloxyethyl-hydroxyethyldimethylammonium, and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably methyl or ethyl halide, a dialkyl sulfate, preferably methyl or ethyl sulfate, methyl methanesulfonate, methyl para-toluenesulfonate, glycol chlorohydrin or glycerol chlorohydrin.

Such compounds are, for example, sold under the names Dehyquart^{®} by Henkel, Stepanquat^{®} by Stepan, Noxamium^{®} by Ceca or Rewoquat^{®} WE 18 by Rewo-Witco.

The composition according to the invention may contain, for example, a mixture of quaternary ammonium monoester, diester and triester salts with a weight majority of diester salts.

Mixtures of ammonium salts that may be used include, for example, the mixture containing 15% to 30% by weight of acyloxy-ethyldihydroxyethylmethylammonium methyl sulfate, 45% to 60% of diacyloxyethylhydroxyethylmethylammonium methyl sulfate and 15% to 30% of triacyloxyethylmethylammonium methyl sulfate, the acyl groups having from 14 to 18 carbon atoms and originating from palm oil, which is optionally partially hydrogenated.

It is also possible to use the ammonium salts containing at least one ester function that are described in patents US-A-4 874 554 and US-A-4 137 180.

Use may be made of behenoylhydroxypropyltrimethyl-ammonium chloride sold by KAO under the name Quartamin BTC 131.

Preferably, the ammonium salts containing at least one ester function contain two ester functions.

Among the cationic surfactants present in the composition according to the invention, it is more particularly preferred to choose cetyltrimethylammonium, behenyltrimethylammonium and dipalrnitoylethylhydroxyethylmethylammonium salts, and mixtures thereof, and more particularly behenyltrimethylammonium chloride, cetyltrimethylammonium chloride, and dipalmitoylethylhydroxyethyl-ammonium methosulfate, and mixtures thereof.

In this first embodiment, the cationic surfactant(s) are present in the composition according to the invention in preferential proportions of at least 0.05% by weight, preferably ranging from 0.05% to 10% by weight, more preferentially from 0.1% to 5% by weight and better still from 0.5% to 5% by weight, relative to the total weight of the composition.

When the cationic surfactant(s) are present in the compositions according to the invention, the weight ratio between the amount of fatty-chain alkoxysilane(s), on the one hand, and the amount of cationic surfactant(s), on the other hand, is preferably greater than or equal to 1

In one preferred embodiment, the weight ratio between the amount of fatty-chain alkoxysilane(s), on the one hand, and the amount of cationic surfactant(s), on the other hand, ranges from 1 to 100, even more preferentially from 1 to 50 and better still from 2 to 20.

According to a first embodiment of the invention, the composition comprises one or more nonionic surfactants.

Examples of nonionic surfactants that may be used in the composition used according to the invention are described, for example, in the "Handbook of Surfactants" by M.R. Porter, published by Blackie & Son (Glasgow and London), 1991, pp. 116-178. They are especially chosen from polyethoxylated, polypropoxylated and/or polyglycerolated alcohols, α-diols and (C₁-C₂₀)alkylphenols, containing at least one fatty chain comprising, for example, from 8 to 18 carbon atoms, the number of ethylene oxide and/or propylene oxide groups possibly ranging especially from 2 to 50, and the number of glycerol groups possibly ranging especially from 2 to 30.

Mention may also be made of copolymers of ethylene oxide and propylene oxide, optionally oxyethylenated fatty acid esters of sorbitan, fatty acid esters of sucrose, polyoxyalkylenated fatty acid esters, optionally oxyalkylenated alkylpolyglycosides, alkylglucoside esters, derivatives of N-alkylglucamine and of N-acylmethylglucamine, aldobionamides and amine oxides.

Unless otherwise mentioned, the term "fatty" compound (for example a fatty acid) denotes a compound comprising, in its main chain, at least one saturated or unsaturated hydrocarbon-based chain, such as alkyl or alkenyl, containing at least 8 carbon atoms, preferably from 8 to 30 carbon atoms, and even better still from 10 to 22 carbon atoms.

The mono- or polyglycosides that may be used in the invention are well known and may be represented more particularly by the general formula (II) below:

R₁O-(R₂O)ₜ (G)ᵥ (VI)

in which:
R₁ represents a linear or branched alkyl and/or alkenyl group, comprising from about 8 to 24 carbon atoms, or an alkylphenyl group whose linear or branched alkyl group comprises from 8 to 24 carbon atoms,
R₂ represents an alkylene group comprising from about 2 to 4 carbon atoms,
G represents a sugar unit comprising from 5 to 6 carbon atoms,
t denotes a value ranging from 0 to 10 and preferably 0 to 4, and
v denotes an integer ranging from 1 to 15.

Mono- or polyglycosides that are preferred in the present invention are (C₈-C₁₈)alkyl mono- or polyglycosides and are compounds of formula (VI) in which:
R₁ more particularly denotes a saturated or unsaturated, linear or branched alkyl group comprising from 8 to 18 carbon atoms,
t denotes a value ranging from 0 to 3 and even more particularly is equal to 0,
G may denote glucose, fructose or galactose, preferably glucose.

The degree of polymerization, i.e. the value of v in formula (VI), may range from 1 to 15 and preferably from 1 to 4. The average degree of polymerization is more particularly between 1 and 2 and even more preferentially from 1.1 to 1.5.

The glycoside bonds between the sugar units are of 1-6 or 1-4 type and preferably of 1-4 type.

Examples of compounds of formula (VI) are especially caprylylglucoside, decylglucoside or caprylglucoside, laurylglucoside, cetearylglucoside and cocoglucoside, and mixtures thereof. These preferred compounds of formula (VI) are especially represented by the products sold by the company Cognis under the names Plantaren^{®} (600 CS/U, 1200 and 2000) or Plantacare^{®} (818, 1200 and 2000). It is also possible to use the products sold by the company SEPPIC under the names Triton CG 110 or Oramix CG 110 and Triton CG 312 or Oramix® NS 10, the products sold by the company BASF under the name Lutensol GD 70 or those sold by the company Chem Y under the name AG 10 LK.

It is also possible to use, for example, (C₈-C₁₆)alkyl-1,4-polyglucoside as an aqueous 53% solution, sold by the company Cognis under the reference Plantacare^{®} 818 UP.

Among the cited nonionic surfactants, optionally oxyalkylenated alkylpolyglycosides are preferably used.

In this second embodiment, the nonionic surfactant(s) are present in the composition according to the invention in preferential proportions of at least 0.1% by weight, preferably ranging from 0.1% to 50% by weight, more preferentially from 1% to 20% by weight and better still from 2% to 10% by weight, relative to the total weight of the composition.

When the nonionic surfactant(s) are present in the composition according to the invention, the weight ratio between the amount of nonionic surfactant(s), on the one hand, and the amount of fatty-chain alkoxysilane(s), on the other hand, is preferably greater than or equal to 0.05.

In one preferred embodiment, the weight ratio between the amount of nonionic surfactant(s), on the one hand, and the amount of fatty-chain alkoxysilane(s), on the other hand, ranges from 0.1 to 50, even more preferentially from 0.15 to 30 and better still from 0.2 to 5.

According to one preferred embodiment, the composition according to the invention also comprises one or more thickeners.

For the purposes of the present invention, the term "thickener" means an agent which, when introduced at 1% by weight in an aqueous solution or an aqueous-alcoholic solution containing 30% ethanol, and at pH 7, makes it possible to achieve a viscosity of at least 100 mPa.s (100 cPs) and preferably of at least 500 mPa.s (500 cPs), at 25°C and at a shear rate of 1 s⁻¹. This viscosity may be measured using a cone/plate viscometer (Haake R600 rheometer or the like).

The thickener or thickeners may be selected from fatty acid amides obtained from C₁₀-C₃₀ carboxylic acid such as monoisopropanolamide, diethanolamide or monoethanolamide of coconut acids, monoethanolamide of ethoxylated carboxylic alkyl ether acid, preferably nonionic cellulose-based thickeners (hydroxyethylcellulose, hydroxypropylcellulose, carboxymethylcellulose), guar gum and its nonionic derivatives such as hydroxypropyl guar, gums of microbial origin such as xanthan gum, scleroglucan gum, crosslinked or non-crosslinked homopolymers and copolymers based on acrylic acid, methacrylic acid or acrylamidopropanesulfonic acid, and associative polymers, especially acrylic associative polymers or polyurethanes, as described below.

The associative polymer(s) that can be used according to the invention are water-soluble polymers which, in an aqueous medium, are capable of reversible association with one another or with other molecules.

Their chemical structure comprises hydrophilic zones and hydrophobic zones characterized by at least one fatty chain preferably comprising from 10 to 30 carbon atoms.

The associative polymer(s) that may be used according to the invention may be of anionic, cationic, amphoteric or nonionic type, for instance the polymers sold under the names Pemulen TR1 or TR2 by the company Goodrich, the INCI name of which is Acrylates/C10-30 Alkyl Acrylate Crosspolymer, Salcare SC90 by the company Ciba, Aculyn 22, 28, 33, 44 or 46 by the company Röhm & Haas, and Elfacos T210 and T212 by the company Akzo.

Among the cited thickeners, use is preferably made of nonionic cellulose-based thickeners, such as hydroxyethylcellulose, associative polyurethanes and xanthan gum.

Preferably, the composition according to the invention comprises from 0.01% to 20% by weight and better still from 0.1% to 10% by weight of thickener(s) relative to the total weight of the composition.

In one particularly advantageous embodiment, the cosmetic composition according to the invention also comprises one or more organic acids.

The term "organic acid" means any non-polymeric organic compound comprising two or more than two carbon atoms and one or more acid functions chosen from carboxylic acid, sulfonic acid and phosphoric acid functions.

Preferentially, the organic acid is not a surfactant.

Even more preferentially, the molecular weight of the organic acid is less than 250 and better still less than 200.

Even more preferentially, the organic acids according to the invention are carboxylic acids and α-hydroxylated carboxylic acids or AHAs.

The organic acids may be amino acids.

The organic acid(s) are preferably chosen from acetic acid, propanoic acid, butanoic acid, lactic acid, malic acid, glycolic acid, ascorbic acid, maleic acid, phthalic acid, succinic acid, taurine, tartaric acid, arginine, glycine, glucuronic acid, gluconic acid and citric acid.

Even more preferentially still, the organic acid used in the composition according to the invention is chosen from acetic acid, citric acid and lactic acid, and is preferably lactic acid.

In the composition, the organic acid(s) may be in free or salified form.

The organic acid(s) that may be used in the composition according to the present invention may be present in a content, expressed as free acids, ranging from 0.01% to 10% by weight, preferably in a content ranging from 0.1% to 8% by weight and even more preferentially in a content ranging from 0.2% to 5% by weight relative to the total weight of the composition.

The composition according to the invention may also comprise one or more additional surfactants chosen from anionic, amphoteric and/or zwitterionic surfactants.

The term "anionic surfactant" means a surfactant comprising, as ionic or ionizable groups, only anionic groups. These anionic groups are preferably chosen from -CO₂H, -CO₂⁻, -SO₃H, -SO₃⁻, -OSO₃H, -OSO₃⁻, -H₂PO₃, -HPO₃⁻, -PO₃²⁻, -H₂PO₂, =HPO₂, -HPO₂⁻, =PO₂⁻, =POH and =PO- groups.

Mention may be made, as examples of anionic surfactants that may be used in the composition according to the invention, of alkyl sulfates, alkyl ether sulfates, alkylamido ether sulfates, alkylaryl polyether sulfates, monoglyceride sulfates, alkylsulfonates, alkylamidesulfonates, alkylarylsulfonates, α-olefin sulfonates, paraffin sulfonates, alkyl sulfosuccinates, alkyl ether sulfosuccinates, alkylamide sulfosuccinates, alkyl sulfoacetates, acylsarcosinates, acylglutamates, alkyl sulfosuccinamates, acylisethionates and N-acyltaurates, polyglycoside polycarboxylic acid and alkyl monoester salts, acyl lactylates, salts of D-galactoside uronic acids, salts of alkyl ether carboxylic acids, salts of alkylaryl ether carboxylic acids, salts of alkylamido ether carboxylic acids; and the corresponding non-salified forms of all these compounds; the alkyl and acyl groups of all these compounds comprising from 6 to 24 carbon atoms and the aryl group denoting a phenyl group.

These compounds may be oxyethylenated and then preferably comprise from 1 to 50 ethylene oxide units.

The salts of C₆-C₂₄ alkyl monoesters of polyglycoside-polycarboxylic acids can be selected from C₆-C₂₄ alkyl polyglycoside-citrates, C₆-C₂₄ alkyl polyglycoside-tartrates and C₆-C₂₄ alkyl polyglycoside-sulfosuccinates.

When the anionic surfactant(s) are in salt form, they may be chosen from alkali metal salts such as the sodium or potassium salt and preferably the sodium salt, the ammonium salts, the amine salts and in particular amino alcohol salts or the alkaline-earth metal salts such as the magnesium salts.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxy-methyl)-aminomethane salts.

Alkali metal or alkaline-earth metal salts, and in particular sodium or magnesium salts, are preferably used.

Among the anionic surfactants mentioned, use is preferably made of (C₆-C₂₄)alkyl sulfates, (C₆-C₂₄)alkyl ether sulfates comprising from 2 to 50 ethylene oxide units, especially in the form of alkali metal, ammonium, amino alcohol and alkaline-earth metal salts, or a mixture of these compounds.

It is particularly preferred to use (C₁₂-C₂₀)alkyl sulfates, (C₁₂-C₂₀)alkyl ether sulfates comprising from 2 to 20 ethylene oxide units, especially in the form of alkali metal, ammonium, amino alcohol and alkaline-earth metal salts, or a mixture of these compounds. Better still, use is made of sodium lauryl ether sulfate containing 2.2 mol of ethylene oxide.

When they are present, the amount of the anionic surfactant(s) preferably ranges from 0.01% to 20% by weight, more preferably from 0.2% to 10% by weight, relative to the total weight of the composition.

The amphoteric or zwitterionic surfactant(s), which are preferably nonsilicone, which can be used in the present invention may especially be derivatives of optionally quaternized aliphatic secondary or tertiary amines, in which derivatives the aliphatic group is a linear or branched chain comprising from 8 to 22 carbon atoms, said amine derivatives containing at least one anionic group, for instance a carboxylate, sulfonate, sulfate, phosphate or phosphonate group. Mention may be made in particular of (C₈-C₂₀)alkylbetaines, sulfobetaines, (C₈-C₂₀)alkylamido(C₃-C₈)alkylbetaines and (C₈-C₂₀)alkylamido(C₆-C₈)alkylsulfobetaines.

Among the optionally quaternized secondary or tertiary aliphatic amine derivatives that can be used, as defined above, mention may also be made of the compounds of respective structures (A1) and (A2) below:

RₐCONHCH₂CH₂N⁺(R_{b})(R_{c})(CH₂COO⁻) (A1)

in which:
Rₐ represents a C₁₀-C₃₀ alkyl or alkenyl group derived from an acid RₐCOOH preferably present in hydrolysed coconut oil, or a heptyl, nonyl or undecyl group;
R_{b} represents a β-hydroxyethyl group, and
R_{c} represents a carboxymethyl group;
   and

   R_{a'}CONHCH₂CH₂N(B)(B') (A2)

   in which:
   B represents the group -CH₂CH₂OX',
   B' represents the group -(CH₂)_{z}Y', with z = 1 or 2,
   X' represents the group -CH₂COOH, CH₂COOZ',
   -CH₂CH₂COOH, -CH₂CH₂COOZ', or a hydrogen atom,
   Y' represents the group -COOH, -COOZ', the group
   -CH₂CHOHSO₃H or the group -CH₂CHOHSO₃Z',
   Z' represents an ion resulting from an alkali metal or alkaline earth metal, such as sodium, an ammonium ion or an ion resulting from an organic amine,
   R_{a'} represents a C₁₀-C₃₀ alkyl or alkenyl group of an acid R_{a'}COOH preferably present in coconut oil or in hydrolysed linseed oil, an alkyl group, especially of C₁₇ and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Among the amphoteric or zwitterionic surfactants mentioned above, use is preferably made of (C₈-C₂₀)alkylbetaines such as cocoylbetaine, and (C₈-C₂₀)alkylamido(C₃-C₈)alkylbetaines such as cocamidopropylbetaine, and mixtures thereof. More preferentially, the amphoteric or zwitterionic surfactant(s) are chosen from cocamidopropylbetaine and cocoylbetaine.

When they are present, the amount of the amphoteric or zwitterionic surfactant(s) preferably ranges from 0.01% to 20% by weight and better still from 0.2% to 10% by weight, relative to the total weight of the composition.

The composition according to the invention also contains one or more alkoxysilanes other than those of formula (I). It may further contain non-siliceous fatty substances, silicones, solid particles, reducing agents and oxidizing agents.

Other alkoxysilanes, other than those of formula (I), are used in the composition and are chosen from the compounds of formula (VII) below: 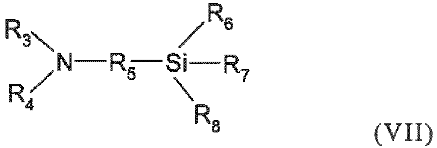 in which:
R₆ represents a halogen or a group OR' or R'₆,
R₇ represents a halogen or a group OR" or R'₇,
R₈ represents a halogen or a group OR''' or R'₈,
R₃, R₄, R₅, R', R", R''', R'₆, R'₇ and R'₈ represent, independently of each other, a saturated or unsaturated, linear or branched hydrocarbon-based group, optionally bearing additional chemical groups, R₃, R₄, R', R" and R''' also possibly denoting hydrogen, at least two of the groups R₆, R₇ and R₈ being different from the groups R'₆, R'₇ and R'₈, at least two of the groups R', R" and R''' being other than hydrogen.

Preferably, the groups R₃, R₄, R', R'₆, R'₇, R'₈, R" and R''' are chosen from C₁-C₁₂ alkyl, C₆-C₁₄ aryl, (C₁-C₈)alkyl(C₆-C₁₄)aryl and (C₆-C₁₄)aryl(C₁-C₈)alkyl radicals.

According to one preferred implementation variant, the alkoxysilane(s) comprise a substituent comprising a primary amine function, and are chosen from the compounds of formula (VIII) below: 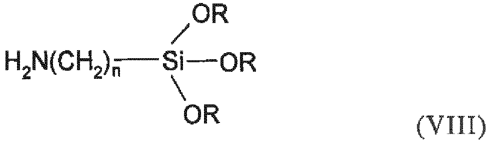 in which the groups R, which may be identical or different, are chosen from C₁-C₆ alkyl groups, and n is an integer from 1 to 6 and preferably from 2 to 4.

One alkoxysilane that is particularly preferred according to this embodiment is γ-aminopropyl triethoxysilane. Such a product is sold, for example, under the name Z-6011 Silane by the company Dow Corning.

According to an even more preferred embodiment, the alkoxysilane(s), other than the alkoxysilanes of the invention, are chosen from the compounds of formula (VIII).

An additional alkoxysilanes that is most particularly preferred is γ-aminopropyltriethoxysilane cited previously.

The alkoxysilane(s), other than the alkoxysilanes of the invention, may be present in the compositions according to the invention in proportions preferably ranging from 0.01% to 25% by weight, more preferentially from 0.05% to 20% by weight and more particularly from 0.1% to 15% by weight relative to the total weight of the composition.

The composition according to the invention may also comprise one or more non-siliceous fatty substances.

The term "fatty substance" means an organic compound that is insoluble in water at standard temperature (25°C) and at atmospheric pressure (760 mmHg, i.e. 1.013×10⁵ Pa), i.e. with a solubility of less than 5%, preferably of less than 1% and even more preferably of less than 0.1%. The non-siliceous fatty substances generally have in their structure a hydrocarbon-based chain comprising at least 6 carbon atoms and not comprising any siloxane groups. In addition, the fatty substances are generally soluble in organic solvents under the same temperature and pressure conditions, for instance chloroform, ethanol, benzene, liquid petroleum jelly or decamethylcyclopentasiloxane.

The term "non-siliceous fatty substance" means a fatty substance whose structure does not comprise any silicon atoms.

The fatty substances that may be used in the composition according to the invention are generally not oxyalkylenated and preferably do not contain any carboxylic acid COOH functions.

Preferably, the non-siliceous fatty substances are chosen from hydrocarbons, fatty alcohols, fatty esters, silicones and fatty ethers, and mixtures thereof.

Even more preferentially, they are chosen from hydrocarbons, fatty alcohols, fatty esters and ceramides, and mixtures thereof.

They may be liquid or non-liquid, at room temperature and at atmospheric pressure.

The liquid fatty substances of the invention preferably have a viscosity of less than or equal to 2 Pa.s, better still less than or equal to 1 Pa.s and even better still less than or equal to 0.1 Pa.s at a temperature of 25°C and at a shear rate of 1 s⁻¹.

The term "liquid hydrocarbon" means a hydrocarbon composed solely of carbon and hydrogen atoms, which is liquid at standard temperature (25°C) and at atmospheric pressure (760 mmHg, i.e. 1.013 × 10⁵ Pa), which is especially of mineral or plant origin, preferably of plant origin.

More particularly, the liquid hydrocarbons are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane,
- linear or branched hydrocarbons of mineral, animal or synthetic origin with more than 16 carbon atoms, such as volatile or non-volatile liquid paraffins, petroleum jelly, liquid petroleum jelly, polydecenes, hydrogenated polyisobutene such as the product sold under the brand name Parleam® by the company NOF Corporation, and squalane.

In one preferred variant, the liquid hydrocarbon(s) are chosen from volatile or non-volatile liquid paraffins, and liquid petroleum jelly.

The term "liquid fatty alcohol" means a non-glycerolated and non-oxyalkylenated fatty alcohol, which is liquid at standard temperature (25°C) and at atmospheric pressure (760 mmHg, i.e. 1.013 × 10⁵ Pa).

Preferably, the liquid fatty alcohols of the invention comprise from 8 to 50 carbon atoms.

The liquid fatty alcohols of the invention may be saturated or unsaturated.

The saturated liquid fatty alcohols are preferably branched. They may optionally comprise in their structure at least one aromatic or non-aromatic ring. They are preferably acyclic.

More particularly, the liquid saturated fatty alcohols of the invention are chosen from octyldodecanol, isostearyl alcohol and 2-hexyldecanol.

Octyldodecanol is most particularly preferred.

The unsaturated liquid fatty alcohols contain in their structure at least one double or triple bond, and preferably one or more double bonds. When several double bonds are present, there are preferably 2 or 3 of them, and they may be conjugated or unconjugated.

These unsaturated fatty alcohols may be linear or branched.

They may optionally comprise in their structure at least one aromatic or non-aromatic ring. They are preferably acyclic.

More particularly, the unsaturated liquid fatty alcohols of the invention are chosen from oleyl alcohol, linoleyl alcohol, linolenyl alcohol and undecylenyl alcohol.

Oleyl alcohol is most particularly preferred.

The term "liquid fatty esters" means an ester derived from a fatty acid and/or from a fatty alcohol that is liquid at standard temperature (25°C) and at atmospheric pressure (760 mmHg, i.e. 1.013 × 10⁵ Pa).

The esters are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isodecyl neopentanoate, isostearyl neopentanoate and isononyl isononanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy alcohols may also be used.

Mention may be made especially of: diethyl sebacate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

The composition may also comprise, as liquid fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include saccharose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be chosen, for example, from oleates, laurates, palmitates, myristates, behenates, cocoates, stearates, linoleates, linolenates, caprates and arachidonates, or mixtures thereof, such as, in particular, oleopalmitate, oleostearate or palmitostearate mixed esters.

More particularly, use is made of monoesters and diesters and in particular of sucrose, glucose or methylglucose mono- or dioleates, stearates, behenates, oleopalmitates, linoleates, linolenates or oleostearates.

An example that may be mentioned is the product sold under the name Glucate® DO by the company Amerchol, which is a methylglucose dioleate.

Finally, use may also be made of natural or synthetic glycerol esters of mono-, di- or triacids.

Among these, mention may be made of plant oils.

As oils of plant origin or synthetic triglycerides that may be used in the composition of the invention as liquid fatty esters, examples that may be mentioned include:
- triglyceride oils of plant or synthetic origin, such as liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, olive oil, rapeseed oil, coconut oil, wheatgerm oil, sweet almond oil, apricot oil, safflower oil, candlenut oil, camellina oil, tamanu oil, babassu oil and pracaxi oil, caprylic/capric acid triglycerides, for instance those sold by the company Stéarineries Dubois or those sold under the names Miglyol^{®} 810, 812 and 818 by the company Dynamit Nobel, jojoba oil and shea butter oil.

Liquid fatty esters derived from monoalcohols will preferably be used as esters according to the invention.

Isopropyl myristate and isopropyl palmitate are particularly preferred.

The liquid fatty ethers are chosen from liquid dialkyl ethers such as dicaprylyl ether.

The fatty substances of the invention may be liquid or non-liquid at room temperature (25°C) and at atmospheric pressure (760 mmHg, i.e. 1.013×10⁵ Pa).

The term "non-liquid" preferably means a solid compound or a compound that has a viscosity of greater than 2 Pa.s at a temperature of 25°C and at a shear rate of 1 s⁻¹.

More particularly, the non-liquid fatty substances are chosen from fatty alcohols, fatty acid and/or fatty alcohol esters, non-siliceous waxes and fatty ethers, which are non-liquid and preferably solid.

The non-liquid fatty alcohols that are suitable for use in the invention are more particularly chosen from saturated or unsaturated, linear or branched alcohols comprising from 8 to 30 carbon atoms. Mention may be made, for example, of cetyl alcohol, stearyl alcohol and a mixture thereof (cetylstearyl alcohol).

As regards the non-liquid esters of fatty acids and/or of fatty alcohols, mention may be made especially of solid esters derived from C₉-C₂₆ fatty acids and from C₉-C₂₆ fatty alcohols.

Among these esters, mention may be made of octyldodecyl behenate, isocetyl behenate, cetyl lactate, stearyl octanoate, octyl octanoate, cetyl octanoate, decyl oleate, myristyl stearate, octyl palmitate, octyl pelargonate, octyl stearate, alkyl myristates such as cetyl myristate, myristyl myristate and stearyl myristate, and hexyl stearate.

Still within the context of this variant, esters of C₄-C₂₂ dicarboxylic or tricarboxylic acids and of C₁-C₂₂ alcohols and esters of monocarboxylic, dicarboxylic or tricarboxylic acids and of C₂-C₂₆ dihydroxy, trihydroxy, tetrahydroxy or pentahydroxy alcohols may also be used.

Mention may be made especially of diethyl sebacate, diisopropyl sebacate, diisopropyl adipate, di-n-propyl adipate, dioctyl adipate and dioctyl maleate.

Among all the esters mentioned above, it is preferred to use myristyl, cetyl or stearyl palmitates, and alkyl myristates such as cetyl myristate, stearyl myristate and myristyl myristate.

The non-siliceous wax(es) are chosen especially from carnauba wax, candelilla wax, esparto wax, paraffin wax, ozokerite, plant waxes, such as olive tree wax, rice wax, hydrogenated jojoba wax or absolute flower waxes, such as the blackcurrant blossom essential wax sold by Bertin (France), or animal waxes, such as beeswaxes or modified beeswaxes (cerabellina), and ceramides.

The ceramides or ceramide analogues, such as glycoceramides, that may be used in the compositions according to the invention are known *per se* and are natural or synthetic molecules that may correspond to the general formula (XIV) below: 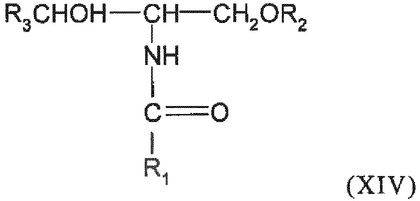 in which:
- R₁ denotes a linear or branched, saturated or unsaturated alkyl group, derived from C₁₄-C₃₀ fatty acids, it being possible for this group to be substituted with a hydroxyl group in the alpha position, or a hydroxyl group in the omega position esterified with a saturated or unsaturated C₁₆-C₃₀ fatty acid,
- R₂ denotes a hydrogen atom or a (glycosyl)ₙ, (galactosyl)ₘ or sulfogalactosyl group, in which n is an integer ranging from 1 to 4 and m is an integer ranging from 1 to 8,
- R₃ denotes a C₁₅-C₂₆ hydrocarbon-based group which is saturated or unsaturated in the alpha position, it being possible for this group to be substituted with one or more C₁-C₁₄ alkyl groups;

it being understood that, in the case of natural ceramides or glycoceramides, R₃ can also denote a C₁₅-C₂₆ α-hydroxyalkyl group, the hydroxyl group being optionally esterified with a C₁₆-C₃₀ α-hydroxy acid.

The ceramides that are preferred in the context of the present invention are those described by Downing in Arch. Dermatol., Vol. 123, 1381-1384, 1987, or those described in French patent FR 2 673 179.

The ceramide(s) that are more particularly preferred according to the invention are the compounds for which R₁ denotes a saturated or unsaturated alkyl derived from C₁₆-C₂₂ fatty acids; R₂ denotes a hydrogen atom; and R₃ denotes a saturated linear C₁₅ group.

Such compounds are, for example, N-linoleyl-dihydrosphingosine, N-oleyl-dihydrosphingosine, N-palmityl-dihydrosphingosine, N-stearyl-dihydrosphingosine or N-behenyldihydrosphingosine, or mixtures of these compounds.

Even more preferentially, use is made of ceramides for which R₁ denotes a saturated or unsaturated alkyl group derived from fatty acids, R₂ denotes a galactosyl or sulfogalactosyl group and R₃ denotes a -CH=CH-(CH₂)₁₂-CH₃ group.

Other waxes or waxy starting materials that may be used according to the invention are especially marine waxes such as those sold by the company Sophim under the reference M82, and waxes of polyethylene or of polyolefins in general.

The non-liquid fatty ethers are chosen from dialkyl ethers and especially dicetyl ether and distearyl ether, alone or as a mixture.

Preferably, the non-siliceous fatty substances according to the invention are chosen from hydrocarbons, fatty alcohols, fatty esters and ceramides.

Even more preferably, the non-siliceous fatty substances are chosen from liquid petroleum jelly, stearyl alcohol, cetyl alcohol and a mixture thereof such as cetylstearyl alcohol, octyldodecanol, oleyl alcohol, isopropyl palmitate, isopropyl myristate, N-oleyldihydrosphingosine, N-behenyldihydrosphingosine and N-linoleyldihydrosphingosine.

The non-siliceous fatty substance(s) may be present in an amount ranging from 0.01% to 40% by weight and especially from 0.1% to 5% by weight relative to the total weight of the composition.

The composition according to the invention may also comprise one or more silicones.

The silicones that may be used in the composition according to the invention are in particular polyorganosiloxanes that may be in the form of aqueous solutions, i.e. dissolved, or optionally in the form of dispersions or microdispersions, or of aqueous emulsions. The polyorganosiloxanes may also be in the form of oils, waxes, resins or gums.

Organopolysiloxanes are defined in greater detail in Walter Noll's Chemistry and Technology of Silicones (1968), Academic Press.

The silicones may be volatile or non-volatile.

When they are volatile, the silicones are more particularly chosen from those with a boiling point of between 60°C and 260°C, and even more particularly from:
(i) cyclic silicones comprising from 3 to 7 and preferably 4 to 5 silicon atoms.
   These are, for example, octamethylcyclotetrasiloxane sold especially under the name Volatile Silicone 7207 by the company Union Carbide or Silbione 70045 V 2 by the company Rhodia, decamethylcyclopentasiloxane sold under the name Volatile Silicone 7158 by the company Union Carbide, and Silbione 70045 V 5 by the company Rhodia, and mixtures thereof.
   Mention may also be made of cyclocopolymers of the dimethylsiloxane/methylalkylsiloxane type, such as Volatile Silicone FZ 3109 sold by the company Union Carbide, of chemical structure: 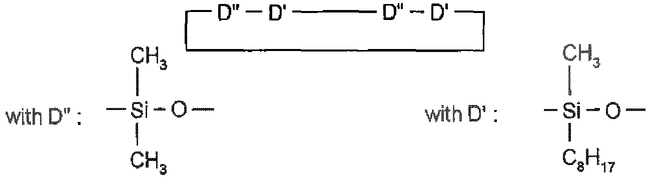
   Mention may also be made of mixtures of cyclic silicones with organosilicon compounds, such as the mixture of octamethyl-cyclotetrasiloxane and tetrakis(trimethylsilyl)-pentaerythritol (50/50) and the mixture of octamethylcyclotetrasiloxane and oxy-1,1'-bis(2,2,2',2',3,3'-hexatrimethyl-silyloxy)neopentane;
(ii) linear volatile silicones containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.*

When the silicones are non-volatile, use is preferably made of polyalkylsiloxanes, polyarylsiloxanes, polyalkylarylsiloxanes, silicone gums and resins, and polyorganosiloxanes modified with organofunctional groups, and mixtures thereof.

These silicones are more particularly chosen from polyalkylsiloxanes, among which mention may be made mainly of polydimethylsiloxanes containing trimethylsilyl end groups (Dimethicone according to the CTFA name) having a viscosity of from 5×10⁻⁶ to 2.5 m²/s at 25°C and preferably 1×10⁻⁵ to 1 m²/s. The viscosity of the silicones is measured, for example, at 25°C according to standard ASTM 445 Appendix C.

Among these polyalkylsiloxanes, mention may be made, in a non-limiting manner, of the following commercial products:
- the Silbione oils of the 47 and 70 047 series or the Mirasil oils sold by the company Rhodia, for instance the oil 70 047 V 500 000,
- the oils of the Mirasil series sold by the company Rhodia,
- the oils of the 200 series from the company Dow Corning, such as, more particularly, DC200 with a viscosity of 60 000 cSt,
- the Viscasil oils from the company General Electric and certain oils of the SF series (SF 96, SF 18) from the company General Electric.

Mention may also be made of polydimethylsiloxanes containing dimethylsilanol end groups (Dimethiconol according to the CTFA name) such as the oils of the 48 series from the company Rhodia.

Mention may also be made of polydimethylsiloxanes containing aminoethyl, aminopropyl and α,ω-silanol groups.

In this category of polyalkylsiloxanes, mention may also be made of the products sold under the names Abil Wax 9800 and 9801 by the company Goldschmidt, which are poly(C₁-C₂₀)alkylsiloxanes.

The polyalkylarylsiloxanes are particularly chosen from linear and/or branched polydimethylmethylphenylsiloxanes and polydimethyldiphenylsiloxanes with a viscosity of from 1×10⁻⁵ to 5×10⁻² m²/s at 25°C.

Among these polyalkylarylsiloxanes, examples that may be mentioned include the products sold under the following names:
- Silbione oils of the 70 641 series from the company Rhodia,
- the oils of the Rhodorsil 70 633 and 763 series from the company Rhodia,
- the oil Dow Corning 556 Cosmetic Grade Fluid from the company Dow Corning,
- silicones of the PK series from the company Bayer, such as the product PK20,
- the silicones of the PN and PH series from the company Bayer, such as the products PN1000 and PH1000,
- certain oils of the SF series from the company General Electric, such as SF 1023, SF 1154, SF 1250 and SF 1265.

The silicone gums that may be present in the composition according to the invention are especially polydiorganosiloxanes having high number-average molecular masses of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Mention may be made more particularly of the following products:
- polydimethylsiloxane gums,
- polydimethylsiloxane/methylvinylsiloxane gums,
- polydimethylsiloxane/diphenylsiloxane gums,
- polydimethylsiloxane/phenylmethylsiloxane gums,
- polydimethylsiloxane/diphenylsiloxane/methylvinylsiloxane gums.

Products that may be used more particularly are the following mixtures:
- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Corning,
- mixtures formed from a polydimethylsiloxane gum with a cyclic silicone, such as the product SF 1214 Silicone Fluid from the company General Electric, this product being an SF 30 gum corresponding to a dimethicone, having a number-average molecular weight of 500 000, dissolved in the oil SF 1202 Silicone Fluid corresponding to decamethylcyclopentasiloxane,
- mixtures of two PDMSs with different viscosities, and more particularly of a PDMS gum and a PDMS oil, such as the product SF 1236 from the company General Electric. The product SF 1236 is a mixture of a gum SE 30 defined above, with a viscosity of 20 m²/s and of an oil SF 96 with a viscosity of 5×10⁻⁶ m²/s. This product preferably comprises 15% of gum SE 30 and 85% of an oil SF 96.

The organopolysiloxane resins that may be present in the composition according to the invention are crosslinked siloxane systems containing the following units: R₂SiO_{2/2}, R₃SiO_{1/2}, RSiO_{3/2} and SiO_{4/2} in which R represents a hydrocarbon group containing 1 to 16 carbon atoms or a phenyl group. Among these products, the ones that are particularly preferred are those in which R denotes a C₁-C₄ alkyl group, more particularly methyl, or a phenyl group.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold in particular under the names X22-4914, X21-5034 and X21-5037 by the company Shin-Etsu.

The organomodified silicones that may be present in the composition according to the invention are silicones as defined above and comprising in their structure one or more organofunctional groups attached via a hydrocarbon-based group.

Among the organomodified silicones, mention may be made of polyorganosiloxanes comprising:
- polyethyleneoxy and/or polypropyleneoxy groups optionally comprising C₆-C₂₄ alkyl groups, such as the products known as dimethicone copolyol sold by the company Dow Corning under the name DC 1248 or the oils Silwet L 722, L 7500, L 77 and L 711 by the company Union Carbide, and the (C₁₂)alkylmethicone copolyol sold by the company Dow Corning under the name Q2 5200,
- thiol groups, such as the products sold under the names GP 72 A and GP 71 from the company Genesee,
- alkoxylated groups, such as the product sold under the name Silicone Copolymer F755 by SWS Silicones and Abil Wax 2428, 2434 and 2440 by the company Goldschmidt,
- hydroxylated groups, such as the polyorganosiloxanes containing a hydroxyalkyl function, described in French patent application FR-A-85/2,589,476,
- acyloxyalkyl groups, for instance the polyorganosiloxanes described in patent US-A-4 957 732,
- anionic groups of the carboxylic acid type, for instance in the products described in patent EP 186 507 from the company Chisso Corporation, or of the alkylcarboxylic type, such as those present in the product X-22-3701E from the company Shin-Etsu; 2-hydroxyalkyl sulfonate; 2-hydroxyalkyl thiosulfate such as the products sold by the company Goldschmidt under the names Abil S201 and Abil S255,
- hydroxyacylamino groups, for instance the polyorganosiloxanes described in patent application EP 342 834. Mention may be made, for example, of the product Q2-8413 from the company Dow Corning.

Among the organomodified silicones, mention may also be made of amino silicones.

For the purposes of the present invention, the term "amino silicone" means any silicone comprising at least one primary, secondary or tertiary amine function or a quaternary ammonium group.

The amino silicones that may be used in the cosmetic composition according to the present invention are chosen from:
(a) the compounds corresponding to formula (XV) below:

   (R₁)ₐ(T)₃₋ₐSi[OSi(T)₂]ₙ[OSi(T)_{b}(R₁)_{2-b}]ₘOSi(T)₃₋ₐ(R₁)ₐ (XV)

   in which:
   T is a hydrogen atom or a phenyl, hydroxyl (-OH) or C₁-C₈ alkyl group, and preferably methyl, or a C₁-C₈ alkoxy, preferably methoxy,
   a denotes the number 0 or an integer from 1 to 3, and preferably 0,
   b denotes 0 or 1, and in particular 1,
   m and n are numbers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10,
   R¹ is a monovalent group of formula -C_{q}H_{2q}L in which q is a number from 2 to 8 and L is an optionally quaternized amino group chosen from the following groups:
      - N(R₂)-CH₂-CH₂-N(R²)₂;
      - N(R²)₂,
      - N⁺(R²)₃Q,
      - N⁺(R²)(H)₂Q⁻,
      - N⁺(R²)₂HQ⁻,
      - N(R²)-CH₂-CH₂-N⁺(R²)(H)₂Q⁻,
   in which R² may denote a hydrogen atom, a phenyl, a benzyl or a saturated monovalent hydrocarbon-based group, for example a C₁-C₂₀ alkyl group, and Q⁻ represents a halide ion, for instance fluoride, chloride, bromide or iodide.

   In particular, the amino silicones corresponding to the definition of formula (XV) are chosen from the compounds corresponding to formula (XVI) below: 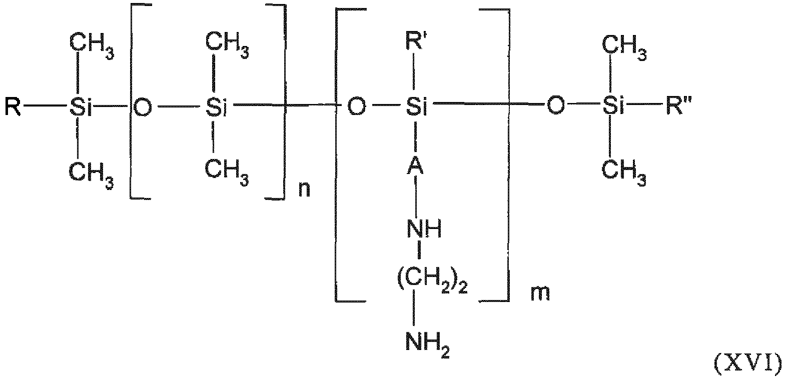 in which R, R' and R", which may be identical or different, denote a C₁-C₄ alkyl group, preferably CH₃; a C₁-C₄ alkoxy group, preferably methoxy; or OH; A represents a linear or branched, C₃-C₈ and preferably C₃-C₆ alkylene group; m and n are integers dependent on the molecular weight and whose sum is between 1 and 2000.
   According to a first possibility, R, R' and R", which may be identical or different, represent a C₁-C₄ alkyl or hydroxyl group, A represents a C₃ alkylene group and m and n are such that the weight-average molecular mass of the compound is between 5000 and 500 000 approximately. Compounds of this type are referred to in the CTFA dictionary as "amodimethicones".
   According to a second possibility, R, R' and R", which may be identical or different, each represent a C₁-C4 alkoxy or hydroxyl group, at least one of the groups R or R" is an alkoxy group and A represents a C₃ alkylene group. The hydroxy/alkoxy mole ratio is preferably between 0.2/1 and 0.4/1 and advantageously equal to 0.3/1. Moreover, m and n are such that the weight-average molecular mass of the compound is between 2000 and 10⁶. More particularly, n is between 0 and 999 and m is between 1 and 1000, the sum of n and m being between 1 and 1000.
   In this category of compounds, mention may be made, inter alia, of the product Belsil® ADM 652 sold by the company Wacker.
   According to a third possibility, R and R", which are different, each represent a C₁-C₄ alkoxy or hydroxyl group, at least one of the groups R or R" being an alkoxy group, R' representing a methyl group and A representing a C₃ alkylene group. The hydroxy/alkoxy mole ratio is preferably between 1/0.8 and 1/1.1 and advantageously equal to 1/0.95. Moreover, m and n are such that the weight-average molecular mass of the compound is between 2000 and 200 000. More particularly, n is between 0 and 999 and m is between 1 and 1000, the sum of n and m being between 1 and 1000.
   More particularly, mention may be made of the product Fluid WR® 1300 sold by the company Wacker.
   Note that the molecular mass of these silicones is determined by gel permeation chromatography (ambient temperature, polystyrene standard; µ styragem columns; eluent THF; flow rate 1 mm/minute; 200 µl of a solution containing 0.5% by weight of silicone in THF are injected, and detection is performed by refractometry and UV-metry).
   A product corresponding to the definition of formula (XV) is in particular the polymer known in the CTFA dictionary as Trimethylsilyl Amodimethicone, corresponding to formula (XVII) below: 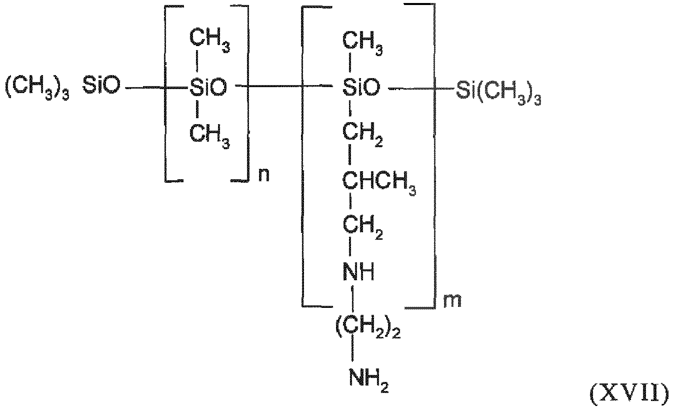 in which n and m have the meanings given above in accordance with formula (XV).
   Such compounds are described, for example, in patent EP 95238. A compound of formula (XVII) is sold, for example, under the name Q2-8220 by the company OSI.
(b) the compounds corresponding to formula (XVIII) below: 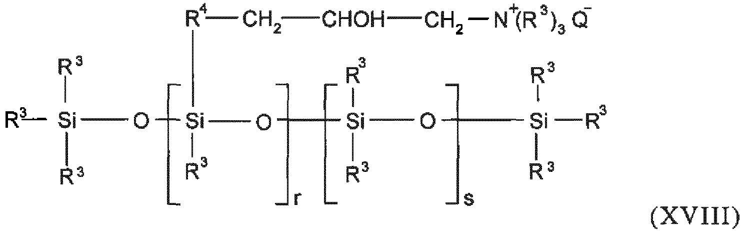 in which:
   R³ represents a C₁-C₁₈ monovalent hydrocarbon-based group, and in particular a C₁-C₁₈ alkyl or C₂-C₁₈ alkenyl group, for example methyl,
   R⁴ represents a divalent hydrocarbon-based group, especially a C₁-C₁₈ alkylene group or a divalent C₁-C₁₈, and for example C₁-C₈, alkylenoxy group,
   Q⁻ is a halide ion, in particular chloride;
   r represents a mean statistical value from 2 to 20 and in particular from 2 to 8,
   s represents a mean statistical value from 20 to 200 and in particular from 20 to 50.

   Such compounds are described more particularly in patent US 4 185 087.
   A compound falling within this class is the product sold by the company Union Carbide under the name Ucar Silicone ALE 56.
(c) quaternary ammonium silicones especially of formula (XIX): 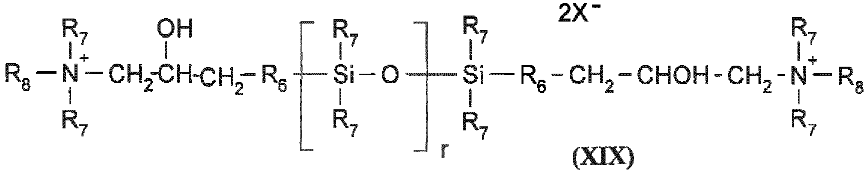 in which:
   R₇, which may be identical or different, represent a monovalent hydrocarbon-based group containing from 1 to 18 carbon atoms, and in particular a C₁-C₁₈ alkyl group, a C₂-C₁₈ alkenyl group or a ring comprising 5 or 6 carbon atoms, for example methyl,
   R₆ represents a divalent hydrocarbon-based group, especially a C₁-C₁₈ alkylene group or a divalent C₁-C₁₈, and for example C₁-C₈, alkylenoxy group linked to the Si via an SiC bond,
   R₈, which may be identical or different, represent a hydrogen atom, a monovalent hydrocarbon-based group containing from 1 to 18 carbon atoms, and in particular a C₁-C₁₈ alkyl group, a C₂-C₁₈ alkenyl group or a group -R₆-NHCOR₇;
   X- is an anion such as a halide ion, especially chloride, or an organic acid salt (acetate, etc.);
   r represents a mean statistical value from 2 to 200 and in particular from 5 to 100.

   These silicones are described, for example, in patent application EP-A-0 530 974.
d) the amino silicones of formula (XX) below: 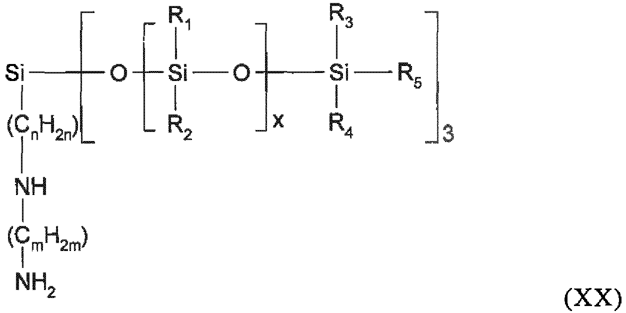 in which:
   - R₁, R₂, R₃ and R₄, which may be identical or different, denote a C₁-C₄ alkyl group or a phenyl group,
   - R₅ denotes a C₁-C₄ alkyl group or a hydroxyl group,
   - n is an integer ranging from 1 to 5,
   - m is an integer ranging from 1 to 5, and
   - x is chosen such that the amine number is between 0.01 and 1 meq/g.

When these compounds are used, one particularly advantageous embodiment involves their combined use with cationic and/or nonionic surfactants.

By way of example, use may be made of the product sold under the name Cationic Emulsion DC939 by the company Dow Corning, a cationic surfactant, namely trimethylcetylammonium chloride and a nonionic surfactant of formula C₁₃H₂₇-(OC₂H₄)₁₂-OH, known under the CTFA name "Trideceth-12".

Another commercial product that may be used according to the invention is the product sold under the name Dow Corning Q2 7224 by the company Dow Corning, comprising, in combination, trimethylsilyl amodimethicone of formula (XVI) described above, a nonionic surfactant of formula C₈H₁₇-C₆H₄-(OCH₂CH₂)₄₀-OH, known under the CTFA name Octoxynol-40, a second nonionic surfactant of formula C₁₂H₂₅-(OCH₂-CH₂)₆-OH, known under the CTFA name Isolaureth-6, and propylene glycol.

The silicones of the invention may also be silicone is grafted with anionic groups, such as the compounds VS 80 or VS 70 sold by the company 3M.

In a more preferred embodiment, the silicone is a chemically unmodified polydimethylsiloxane.

The silicone(s) may be present in contents ranging from 0.01% to 40% by weight and preferably from 0.1% to 5% by weight relative to the total weight of the composition.

The composition may also comprise one or more solid particles. Among these solid particles, antidandruff agents such as zinc pyridinethione, selenium disulfide and ellagic acid, fillers, and especially silica, titanium dioxide, pigments, dyes, abrasive powders such as pumice and apricot kernel powder may be present in the composition according to the invention.

The composition according to the present invention may also contain one or more reducing agents, especially such as sulfureous reducing agents. These agents are preferably chosen from organic compounds comprising one or more mercapto groups (-SH), sulfites and sulfite derivatives.

The term "sulfite derivatives" essentially denotes bisulfites and sulfite diesters of formula ROSO₂R', with R and R' denoting C₁-C₁₀ alkyl groups.

The organic compounds comprising a mercapto group are preferably chosen from the following compounds: thioglycolic acid, thiolactic acid, cysteine, homocysteine, glutathione, thioglycerol, thiomalic acid, 2-mercaptopropionic acid, 3-mercaptopropionic acid, thiodiglycol, 2-mercaptoethanol, dithiothreitol, thioxanthine, thiosalicylic acid, thiodiglycolic acid, lipoic acid, N-acetylcysteine, and thioglycolic or thiolactic acid esters, and mixtures of these compounds.

The sulfureous reducing agent(s) may be used especially in the form of salts, in particular alkali metal salts such as sodium and potassium salts, alkaline-earth metal salts, for example magnesium and calcium salts, ammonium salts, amine salts and amino alcohol salts.

In a particularly preferred manner, the sulfureous reducing agent(s) are chosen from thioglycolic acid and salts thereof, thiolactic acid and salts thereof, alkali metal sulfites and especially sodium sulfite, alkali metal bisulfites and especially sodium bisulfite, and precursors of these sulfites or bisulfites such as sodium metabisulfite.

The sulfureous reducing agent(s) may be present in an amount ranging from 0.1% to 5% by weight and especially from 0.3% to 3% by weight relative to the total weight of the composition.

The composition in accordance with the invention may also comprise at least one oxidizing agent.

Such an oxidizing agent is preferably chosen from the group formed by hydrogen peroxide, urea peroxide, alkali metal bromates or ferricyanides, and persalts such as perborates and persulfates.

The use of hydrogen peroxide is particularly preferred.

According to one preferred embodiment, the composition according to the invention may also comprise one or more cationic polymers.

The composition according to the invention may be aqueous or anhydrous.

The term "anhydrous" refers to a composition not containing any added water, i.e. a composition in which the water that may be present comes only from the water of crystallization or of adsorption of the starting materials. In any case, an anhydrous composition contains less than 5% by weight of water and preferably less than 1% by weight of water relative to the total weight of the composition.

Whether it is anhydrous or aqueous, the composition according to the invention may contain one or more organic solvents that are liquid at room temperature (25°C) and at atmospheric pressure (760 mmHg, i.e. 1.013 × 10⁵ Pa). Preferably, the liquid organic solvent(s) are chosen from C₁-C₄ lower alcohols, such as ethanol, isopropanol, tert-butanol or n-butanol, polyols such as propylene glycol, polyol ethers, C₅-C₁₀ alkanes, C₃-C₄ ketones such as acetone and methyl ethyl ketone, C₁-C₄ alkyl acetates such as methyl acetate, ethyl acetate and butyl acetate, dimethoxyethane, diethoxyethane, silicone oils and the non-siliceous liquid fatty substances described above, and mixtures thereof.

When the composition of the invention is aqueous, its pH is generally between 2 and 9 and in particular between 3 and 8. Preferably, the pH is less than 7. Even more preferentially, it ranges from 3 to 6.

It may be adjusted to the desired value by means of acidifying or basifying agents usually used in cosmetics for this type of application, or alternatively using standard buffer systems.

Among the acidifying agents, examples that may be mentioned include the organic acids already mentioned previously, or mineral acids.

The term "mineral acid" means any acid derived from a mineral compound. Among the mineral acids, mention may be made of hydrochloric acid, orthophosphoric acid, sulfuric acid, sulfonic acids and nitric acid.

Use may be made especially of mineral or organic acids such as hydrochloric acid, orthophosphoric acid, sulfuric acid, carboxylic acids, for instance acetic acid, tartaric acid, citric acid or lactic acid, and sulfonic acids.

Among the basifying agents, examples that may be mentioned include aqueous ammonia, alkali metal carbonates, alkanolamines, such as mono-, di- and triethanolamines and derivatives thereof, sodium hydroxide, potassium hydroxide and the compounds of the following formula: 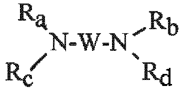 in which W is a propylene residue optionally substituted with a hydroxyl group or a C₁-C₄ alkyl group; Rₐ, R_{b}, R_{c} and R_{d}, which may be identical or different, represent a hydrogen atom or a C₁-C₄ alkyl or C₁-C₄ hydroxyalkyl group.

Preferably, the pH modifiers may be chosen from alkaline agents such as aqueous ammonia, monoethanolamine, diethanolamine, triethanolamine, 1,3-propanediamine or an alkaline hydroxide, such as 2-amino-2-methyl-1-propanol, or from acidifying agents such as phosphoric acid or hydrochloric acid.

The compositions according to the invention may also comprise one or more additives chosen from fixing polymers, pseudoceramides, vitamins and provitamins, including panthenol, water-soluble or liposoluble, silicone or non-silicone sunscreens, nacreous agents and opacifiers, sequestrants, conditioning agents other than the silicones and cationic polymers mentioned previously, solubilizers, antioxidants, antidandruff agents other than those mentioned above, anti-seborrhoeic agents, hair-loss counteractants and/or hair restorers, penetrants, fragrances, peptizers and preserving agents, or any other additive conventionally used in the cosmetics field.

These additives can be present in the composition according to the invention in an amount ranging from 0 to 20% by weight, with respect to the total weight of the composition.

A person skilled in the art will take care to select the optional additives and the amounts thereof such that they do not harm the properties of the compositions of the present invention.

The present invention also relates to a cosmetic process for the treatment of keratin materials, which consists in applying to said materials an effective amount of a composition as described above.

This application may or may not be followed by a rinsing operation.

When the application of the composition is followed by rinsing, the leave-on time of the composition on the keratin materials ranges from a few seconds to 60 minutes, better still from 5 seconds to 30 minutes, even better still from 10 seconds to 10 minutes.

Whether in rinse-out mode or leave-in mode, the application of the composition may take place in the presence or absence of heat especially in the case of hair-care. The heating device may be a hairdryer, a hood dryer, a curling iron or a flat iron. The heating temperature may be between 40°C and 220°C.

The application of the composition according to the invention to the hair may take place on dry hair or on wet hair. It may in particular be carried out after a shampooing operation or after a pretreatment at acidic or basic pH.

## Claims

1. Cosmetic composition comprising:
- at least 0.1% by weight, relative to the total weight of the composition, of one or more fatty-chain alkoxysilanes of formula (I) below:
RiSi(OR₂)₃ (I)
in which R₁ represents a linear or branched alkyl or alkenyl group comprising from 7 to 18 carbon atoms, and R₂ represents a linear or branched alkyl group comprising from 1 to 6 carbon atoms,
- one or more surfactant(s) chosen from cationic surfactants and nonionic surfactants, and mixtures thereof, and
- one or more alkoxysilane(s), other than the alkoxysilanes of formula (I), chosen from compounds of formula (VII) below: 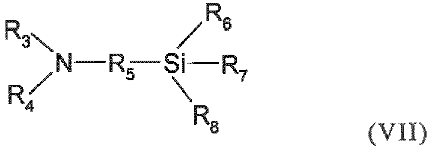 in which:
R₆ represents a halogen or a group OR' or R'₆,
R₇ represents a halogen or a group OR" or R'₇,
R₈ represents a halogen or a group OR''' or R'₈, R₃, R₄, R₅, R', R", R''', R'₆, R'₇ and R'₈ represent, independently of each other, a saturated or unsaturated, linear or branched hydrocarbon-based group, optionally bearing additional chemical groups, R₃, R₄, R', R" and R''' also possibly denoting hydrogen, at least two of the groups R₆, R₇ and R₈ being different from the groups R'₆, R'₇ and R'₈, at least two of the groups R', R" and R''' being other than hydrogen.

2. Composition according to Claim 1, **characterized in that** R₂ represents an alkyl group comprising from 1 to 4 carbon atoms.

3. Composition according to Claim 1 or 2, **characterized in that** R₁ represents an alkyl group.

4. Composition according to any one of Claims 1 to 3, **characterized in that** the alkoxysilane of formula (I) is chosen from octyltriethoxysilane, dodecyltriethoxysilane, octadecyltriethoxysilane and hexadecyltriethoxysilane.

5. Composition according to any one of the preceding claims, **characterized in that** the alkoxysilane(s) of formula (I) are present in proportions ranging from 0.1% to 20% by weight, relative to the total weight of the composition.

6. Composition according to any one of the preceding claims, **characterized in that** the cationic surfactant(s) are chosen from:
a) quaternary ammonium salts of formula (II) below: 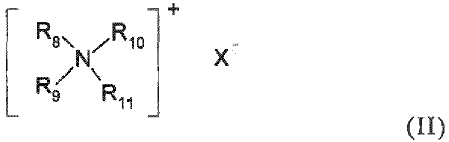 in which the groups R₈ to R₁₁, which may be identical or different, represent a linear or branched aliphatic group comprising from 1 to 30 carbon atoms or an aromatic group such as aryl or alkylaryl, at least one of the groups R₈ to R₁₁ comprising from 8 to 30 carbon atoms;
b) quaternary ammonium salts of imidazoline of formula (III) below: 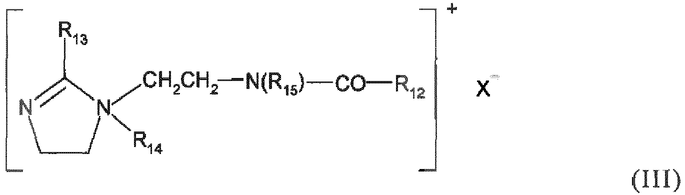 in which R₁₂ represents an alkyl or alkenyl group comprising from 8 to 30 carbon atoms, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X⁻ is an anion chosen from the group of halides, phosphates, acetates, lactates, (C₁-C₄)alkyl sulfates and (C₁-C₄)alkyl- or (C₁-C₄)alkylaryl-sulfonates;
c) quaternary diammonium or triammonium salts, particularly of formula (IV) below: 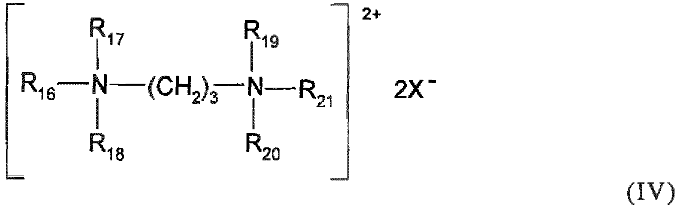 in which R₁₆ denotes an alkyl group comprising about 16 to 30 carbon atoms, optionally hydroxylated and/or interrupted with one or more oxygen atoms; R₁₇ is chosen from hydrogen, an alkyl group comprising from 1 to 4 carbon atoms or a group -(CH₂)₃-N⁺(R₁₆ₐ)(R₁₇ₐ)(R₁₈ₐ); R₁₆ₐ, R₁₇ₐ, R₁₈ₐ, R₁₈, R₁₉, R₂₀ and R₂₁, which may be identical or different, are chosen from hydrogen and an alkyl group comprising from 1 to 4 carbon atoms, and X⁻ is an anion chosen from the group of halides, acetates, phosphates, nitrates, (C₁-C₄)alkyl sulfates and (C₁-C₄)alkyl- or (C₁-C₄)alkylaryl-sulfonates, in particular methyl sulfate and ethyl sulphate;
d) quaternary ammonium salts comprising one or more ester functional groups, such as those of following formula (V): 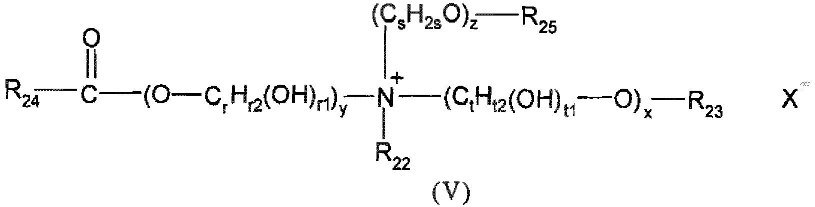 in which:
R₂₂ is chosen from C₁-C₆ alkyl and C₁-C₆ hydroxyalkyl or dihydroxyalkyl groups;
R₂₃ is chosen from the group: 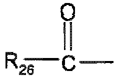
- linear or branched, saturated or unsaturated C₁-C₂₂ hydrocarbon-based groups R₂₇,
- a hydrogen atom,
R₂₅ is chosen from:
- the group 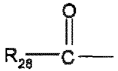
- linear or branched, saturated or unsaturated C₁-C₆ hydrocarbon-based groups R₂₉,
- a hydrogen atom,
R₂₄, R₂₆ and R₂₈, which may be identical or different, are chosen from linear or branched, saturated or unsaturated C₇-C₂₁ hydrocarbon-based groups;
r, s and t, which may be identical or different, are integers ranging from 2 to 6,
r1 and t1, which may be identical or different, are equal to 0 or 1, r2+ r1 = 2r and t1 + t2 = 2t,
y is an integer ranging from 1 to 10,
x and z, which may be identical or different, are integers ranging from 0 to 10,
X- is a simple or complex, organic or mineral anion,
with the proviso that the sum x + y + z is from 1 to 15, that when x is 0, then R₂₃ denotes R₂₇ and that when z is 0, then R₂₅ denotes R₂₉.

7. Composition according to any one of the preceding claims, **characterized in that** the cationic surfactant(s) are present in proportions of at least 0.05% by weight relative to the total weight of the composition.

8. Composition according to any one of the preceding claims, **characterized in that** the nonionic surfactant(s) are chosen from:
- polyethoxylated, polypropoxylated and/or polyglycerolated alcohols, α-diols and (C₁-C₂₀)alkylphenols, containing at least one fatty chain comprising from 8 to 18 carbon atoms,
- copolymers of ethylene oxide and propylene oxide, optionally oxyethylenated fatty acid esters of sorbitan, fatty acid esters of sucrose, polyoxyalkylenated fatty acid esters, optionally oxyalkylenated alkylpolyglycosides, alkylglucoside esters, aldobionamides and amine oxides.

9. Composition according to any one of the preceding claims, **characterized in that** the nonionic surfactant(s) are present in proportions of at least 0.1% by weight, relative to the total weight of the composition.

10. Composition according to any one of the preceding claims, **characterized in that** it contains one or more cationic surfactant(s), with a weight ratio between the amount of fatty-chain alkoxysilane(s), on the one hand, and the amount of cationic surfactant(s), on the other hand, greater than or equal to 1.

11. Composition according to any one of the preceding claims, **characterized in that** it contains one or more nonionic surfactant(s), with a weight ratio between the amount of nonionic surfactant(s), on the one hand, and the amount of fatty-chain alkoxysilane(s), on the other hand, greater than or equal to 0.05.

12. Composition according to any one of the preceding claims, **characterized in that** it also comprises one or more thickeners, present in a content ranging from 0.01% to 20% by weight relative to the total weight of the composition.

13. Composition according to any one of the preceding claims, **characterized in that** it also comprises one or more organic acids chosen from acetic acid, propanoic acid, butanoic acid, lactic acid, malic acid, glycolic acid, ascorbic acid, maleic acid, phthalic acid, succinic acid, taurine, tartaric acid, glycine, glucuronic acid, gluconic acid and citric acid.

14. Composition according to any one of the preceding claims, **characterized in that** it also comprises one or more additives chosen from non-siliceous fatty substances, silicones, solid particles, reducing agents and oxidizing agents.

15. Composition according to any one of the preceding claims, **characterized in that** it is aqueous.

16. Composition according to any one of Claims 1 to 14, **characterized in that** it is anhydrous.

17. Use of a composition according to any one of claims 1 to 16 for treating keratin materials, **characterized in that** it consists in applying to the said materials an effective amount of a composition according to any one of Claims 1 to 16 and then in optionally rinsing it out after an optional leave-on time, in the presence or absence of heat.

## Patentansprüche

1. Kosmetische Zusammensetzung, umfassend:
- mindestens 0,1 Gew.-%, bezogen auf das Gesamtgewicht der Zusammensetzung, eines oder mehrerer Fettkettenalkoxysilane der nachstehenden Formel (I):
R₁Si(OR₂)₃ (I)
in der R₁ für eine gerade oder verzweigte Alkyl- oder Alkenylgruppe mit 7 bis 18 Kohlenstoffatomen steht und R₂ für eine lineare oder verzweigte Alkylgruppe mit 1 bis 6 Kohlenstoffatomen steht,
- ein oder mehrere Tenside, die aus kationischen Tensiden und nichtionischen Tensiden und Mischungen davon ausgewählt sind, und
- ein oder mehrere Alkoxysilane, die von den Alkoxysilanen der Formel (I) verschieden sind, ausgewählt aus Verbindungen der nachstehenden Formel (VII): 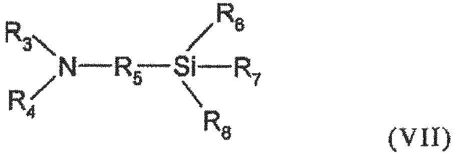 in der:
R₆ für ein Halogen oder eine Gruppe O-R' oder R'₆ steht,
R₇ für ein Halogen oder eine Gruppe O-R" oder R'₇ steht,
R₈ für ein Halogen oder eine Gruppe O-R''' oder R'₈ steht,
R₃, R₄, R₅, R', R", R''', R'₆, R'₇ und R'₈ unabhängig voneinander für eine gesättigte oder ungesättigte, lineare oder verzweigte kohlenwasserstoffbasierte Gruppe stehen, die gegebenenfalls zusätzliche chemische Gruppen trägt, wobei R₃, R₄, R', R" und R''' auch Wasserstoff bedeuten können, wobei mindestens zwei der Gruppen R₆, R₇ und R₈ von den Gruppen R'₆, R'₇ und R'₈ verschieden sind und mindestens zwei der Gruppen R', R" und R''' von Wasserstoff verschieden sind.

2. Zusammensetzung nach Anspruch 1, **dadurch gekennzeichnet, dass** R₂ für eine Alkylgruppe mit 1 bis 4 Kohlenstoffatomen steht.

3. Zusammensetzung nach Anspruch 1 oder 2, **dadurch gekennzeichnet, dass** R₁ für eine Alkylgruppe steht.

4. Zusammensetzung nach einem der Ansprüche 1 bis 3, **dadurch gekennzeichnet, dass** das Alkoxysilan der Formel (I) aus Octyltriethoxysilan, Dodecyltriethoxysilan, Octadecyltriethoxysilan und Hexadecyltriethoxysilane ausgewählt ist.

5. Zusammensetzung nach einem der vorhergehenden Ansprüche, **dadurch gekennzeichnet, dass** das Alkoxysilan bzw. die Alkoxysilane der Formel (I) in Anteilen im Bereich von 0,1 bis 20 Gew.-%, bezogen auf das Gesamtgewicht der Zusammensetzung, vorliegt bzw. vorliegen.

6. Zusammensetzung nach einem der vorhergehenden Ansprüche, **dadurch gekennzeichnet, dass** das kationische Tensid bzw. die kationischen Tenside aus:
a) quaternären Ammoniumsalzen der nachstehenden Formel (II): 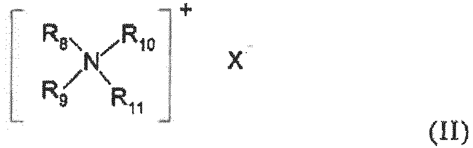 in der die Gruppen R₈ bis R₁₁, die gleich oder verschieden sein können, für eine lineare oder verzweigte aliphatische Gruppe mit 1 bis 30 Kohlenstoffatomen oder eine aromatische Gruppe wie Aryl oder Alkylaryl stehen, wobei mindestens eine der Gruppen R₈ bis R₁₁, 8 bis 30 Kohlenstoffatome umfasst,
b) quaternären Ammoniumsalzen von Imidazolin der nachstehenden Formel (III): 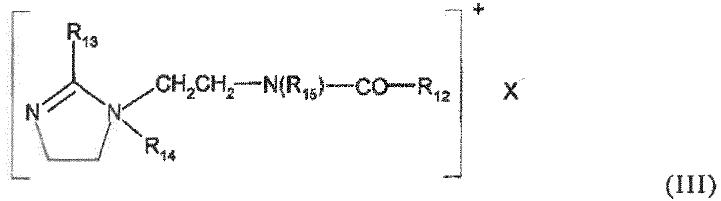 in der R₁₂ für eine Alkyl- oder Alkenylgruppe mit 8 bis 30 Kohlenstoffatomen steht, R₁₃ für ein Wasserstoffatom, eine C₁-C₄-Alkylgruppe oder eine Alkyl- oder Alkenylgruppe mit 8 bis 30 Kohlenstoffatomen steht, R₁₄ für eine C₁-C₄-Alkylgruppe steht, R₁₅ für ein Wasserstoffatom oder eine C₁-C₄-Alkylgruppe steht, X⁻ für ein Anion aus der Gruppe der Halogenide, Phosphate, Acetate, Lactate, (C₁-C₄)-Alkylsulfate und (C₁-C₄) -Alkyl- oder (C₁-C₄)-Alkylarylsulfonate steht;
c) quaternären Diammonium- oder Triammoniumsalzen, insbesondere der nachstehenden Formel (IV): 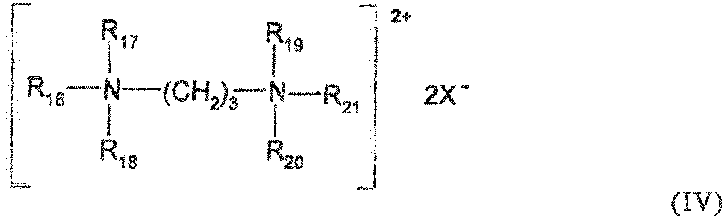 in der R₁₆ für eine Alkylgruppe mit etwa 16 bis 30 Kohlenstoffatomen, die gegebenenfalls hydroxyliert und/oder durch ein oder mehrere Sauerstoffatome unterbrochen ist, steht; R₁₇ aus Wasserstoff, einer Alkylgruppe mit 1 bis 4 Kohlenstoffatomen oder einer Gruppe - (CH₂)₃-N⁺(R₁₆ₐ) (R₁₇ₐ) (R₁₈ₐ) ausgewählt ist; R,₆ₐ, R₁₇ₐ, R₁₈ₐ, R₁₈, R₁₉, R₂₀ und R₂₁, die gleich oder verschieden sein können, aus Wasserstoff und einer Alkylgruppe mit 1 bis 4 Kohlenstoffatomen ausgewählt sind und X⁻ für ein Anion aus der Gruppe der Halogenide, Acetate, Phosphate, Nitrate, (C₁-C₄)-Alkylsulfate und (C₁-C₄) -Alkyl- oder (C₁-C₄) - Alkylarylsulfonate, insbesondere Methylsulfat und Ethylsulfat, steht;
d) quaternären Ammoniumsalzen mit einer oder mehreren Esterfunktionen, wie denjenigen der folgenden Formel (V): 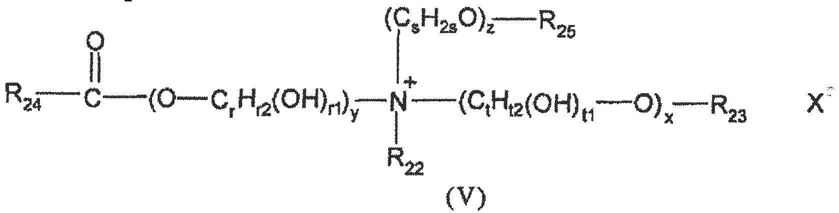 in der:
R₂₂ aus C₁-C₆-Alkyl- und C₁-C₆-Hydroxyalkyl- oder Dihydroxyalkylgruppen ausgewählt ist;
R₂₃ aus der Gruppe: 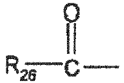
- linearen oder verzweigten, gesättigten oder ungesättigten C₁-C₂₂-kohlenwasserstoffbasierten Gruppen R₂₇,
- einem Wasserstoffatom
ausgewählt ist;
R₂₅ aus:
- der Gruppe 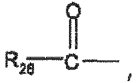
- linearen oder verzweigten, gesättigten oder ungesättigten C₁-C₆-kohlenwasserstoffbasierten Gruppen R₂₉,
- einem Wasserstoffatom
ausgewählt ist;
R₂₄, R₂₆ und R₂₈, die gleich oder verschieden sein können, aus linearen oder verzweigten, gesättigten oder ungesättigten C₇-C₂₁-kohlenwasserstoffbasierten Gruppen ausgewählt sind;
r, s und t, die gleich oder verschieden sein können, ganze Zahlen im Bereich von 2 bis 6 sind,
r1 und t1, die gleich oder verschieden sein können, gleich 0 oder 1 sind,
r2 + r1 = 2 r und t1 + t2 = 2 t,
y eine ganze Zahl im Bereich von 1 bis 10 ist,
x und z, die gleich oder verschieden sein können, ganze Zahlen im Bereich von 0 bis 10 sind,
X⁻ für ein einfaches oder komplexes, organisches oder mineralisches Anion steht,
mit der Maßgabe, dass die Summe von x + y + z 1 bis 15 beträgt, dass dann, wenn x gleich 0 ist, R₂₃ für R₂₇ steht, und dann, wenn z gleich 0 ist, R₂₅ für R₂₉ steht,
ausgewählt ist bzw. sind.

7. Zusammensetzung nach einem der vorhergehenden Ansprüche, **dadurch gekennzeichnet, dass** das kationische Tensid bzw. die kationischen Tenside in Anteilen von mindestens 0,05 Gew.-%, bezogen auf das Gesamtgewicht der Zusammensetzung, vorliegt bzw. vorliegen.

8. Zusammensetzung nach einem der vorhergehenden Ansprüche, **dadurch gekennzeichnet, dass** das nichtionische Tensid bzw. die nichtionischen Tenside aus:
- polyethoxylierten, polypropoxylierten und/oder polyglycerinierten Alkoholen, α-Diolen und (C₁-C₂₀)-Alkylphenolen, die mindestens eine Fettkette mit 8 bis 18 Kohlenstoffatomen enthalten,
- Copolymeren von Ethylenoxid und Propylenoxid, gegebenenfalls oxyethylenierten Fettsäureestern von Sorbitan, Fettsäureestern von Saccharose, polyoxyalkylenierten Fettsäureestern, gegebenenfalls oxyalkylenierten Alkylpolyglykosiden, Alkylglucosidestern, Aldobionamiden und Aminoxiden ausgewählt ist bzw. sind.

9. Zusammensetzung nach einem der vorhergehenden Ansprüche, **dadurch gekennzeichnet, dass** das nichtionische Tensid bzw. die nichtionischen Tenside in Anteilen von mindestens 0,1 Ges.-%, bezogen auf das Gesamtgewicht der Zusammensetzung, vorliegt bzw. vorliegen.

10. Zusammensetzung nach einem der vorhergehenden Ansprüche, **dadurch gekennzeichnet, dass** sie ein oder mehrere kationische Tenside enthält, wobei das Gewichtsverhältnis zwischen der Menge an Fettkettenalkoxysilan(en) einerseits und der Menge an kationischem Tensid bzw. kationischen Tensiden andererseits größer gleich 1 ist.

11. Zusammensetzung nach einem der vorhergehenden Ansprüche, **dadurch gekennzeichnet, dass** sie ein oder mehrere nichtionische Tenside enthält, wobei das Gewichtsverhältnis zwischen der Menge an nichtionischem Tensid bzw. nichtionischen Tensiden einerseits und der Menge an Fettkettenalkoxysilan(en) andererseits größer gleich 0,05 ist.

12. Zusammensetzung nach einem der vorhergehenden Ansprüche, **dadurch gekennzeichnet, dass** sie außerdem einen oder mehrere Verdicker enthält, die in einem Gehalt im Bereich von 0,01 bis 20 Gew.-%, bezogen auf das Gesamtgewicht der Zusammensetzung, vorliegen.

13. Zusammensetzung nach einem der vorhergehenden Ansprüche, **dadurch gekennzeichnet, dass** sie außerdem eine oder mehrere organische Säuren, die aus Essigsäure, Propansäure, Butansäure, Milchsäure, Äpfelsäure, Glykolsäure, Ascorbinsäure, Maleinsäure, Phthalsäure, Bernsteinsäure, Taurin, Weinsäure, Glycin, Glucuronsäure, Gluconsäure und Citronensäure ausgewählt sind, umfasst.

14. Zusammensetzung nach einem der vorhergehenden Ansprüche, **dadurch gekennzeichnet, dass** sie außerdem ein oder mehrere Additive, die aus nicht siliciumhaltigen Fettsubstanzen, Silikonen, festen Teilchen, Reduktionsmitteln und Oxidationsmitteln ausgewählt sind, umfasst.

15. Zusammensetzung nach einem der vorhergehenden Ansprüche, **dadurch gekennzeichnet, dass** sie wässrig ist.

16. Zusammensetzung nach einem der Ansprüche 1 bis 14, **dadurch gekennzeichnet, dass** sie wasserfrei ist.

17. Verwendung einer Zusammensetzung nach einem der Ansprüche 1 bis 16 zur Behandlung von Keratinmaterialien, **dadurch gekennzeichnet, dass** sie darin besteht, dass man auf die Materialien eine wirksame Menge einer Zusammensetzung nach einem der Ansprüche 1 bis 16 aufbringt und sie dann nach einer fakultativen Einwirkungszeit in An- oder Abwesenheit von Wärme fakultativ ausspült.

## Revendications

1. Composition cosmétique comprenant :
- au moins 0,1 %, en poids rapporté au poids total de la composition, d'un ou de plusieurs alcoxy-silane(s) à chaîne grasse, de formule (I) suivante :
R₁Si(OR₂)₃ (I)
dans laquelle R₁ représente un groupe alkyle ou alcényle, linéaire ou ramifié, comportant de 7 à 18 atomes de carbone, et R₂ représente un groupe alkyle, linéaire ou ramifié, comportant de 1 à 6 atomes de carbone,
- un ou plusieurs tensioactif(s) choisi(s) parmi les tensioactifs cationiques, les tensioactifs non-ioniques, et les mélanges de tels tensioactifs,
- et un ou plusieurs alcoxy-silane(s) autre(s) que les alcoxy-silanes de formule (I), choisi(s) parmi les composés de formule (VII) suivante : 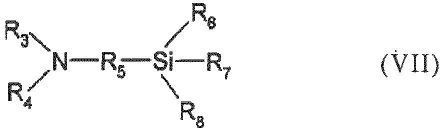 dans laquelle
R₆ représente un atome d'halogène ou un groupe symbolisé par OR' ou par R'₆,
R₇ représente un atome d'halogène ou un groupe symbolisé par OR" ou par R'₇,
R₈ représente un atome d'halogène ou un groupe symbolisé par OR''' ou par R'₈,
et chacun des symboles R₃, R₄, R₅, R', R", R''', R'₆, R'₇ et R'₈ représente, indépendamment des autres, un groupe hydrocarboné linéaire ou ramifié, saturé ou insaturé, en option porteur de groupes chimiques supplémentaires, étant entendu que R₃, R₄, R', R" et R''' peuvent aussi représenter chacun un atome d'hydrogène, qu'au moins deux des groupes symbolisés par R₆, R₇ et R₈ diffèrent de ceux symbolisés par R'₆, R'₇ et R'₈, et qu'au moins deux des symboles R', R" et R''' représentent autre chose qu'un atome d'hydrogène.

2. Composition conforme à la revendication 1, **caractérisée en ce que** R₂ représente un groupe alkyle comportant de 1 à 4 atomes de carbone.

3. Composition conforme à la revendication 1 ou 2, **caractérisée en ce que** R₁ représente un groupe alkyle.

4. Composition conforme à l'une des revendications 1 à 3, **caractérisée en ce que** l'alcoxy-silane de formule (I) est choisi parmi les octyl-triéthoxysilane, dodécyl-triéthoxysilane, octadécyl-triéthoxysilane et hexadécyl-triéthoxysilane,

5. Composition conforme à l'une des revendications précédentes, **caractérisée en ce que** le ou les alcoxy-silane(s) de formule (I) s'y trouve(nt) présent(s) en une proportion valant de 0,1 à 20 %, en poids rapporté au poids total de la composition.

6. Composition conforme à l'une des revendications précédentes, **caractérisée en ce que** le ou les tensioactif(s) cationique(s) est ou sont choisi(s) parmi les suivants :
a) les sels d'ammonium quaternaire de formule (II) suivante : 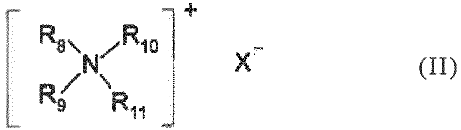 dans laquelle les entités symbolisées par R₈ à R₁₁, qui peuvent être identiques ou différentes, sont des groupes aliphatiques, linéaires ou ramifiés, comportant de 1 à 30 atomes de carbone, ou des groupes aromatiques comme des groupes aryle ou alkyl-aryle, étant entendu qu'au moins l'un des groupes symbolisés par R₈ à R₁₁ comporte de 8 à 30 atomes de carbone ;
b) les sels d'ammonium quaternaire d'imidazoline, de formule (III) suivante : 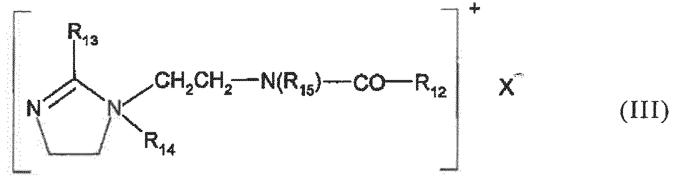 dans laquelle R₁₂ représente un groupe alkyle ou alcényle, comportant de 8 à 30 atomes de carbone, R₁₃ représente un atome d'hydrogène, un groupe alkyle en C₁-C₄, ou un groupe alkyle ou alcényle, comportant de 8 à 30 atomes de carbone, R₁₄ représente un groupe alkyle en C₁-C₄, R₁₅ représente un atome d'hydrogène ou un groupe alkyle en C₁-C₄, et X- représente un anion choisi dans l'ensemble constitué par les anions halogénures, phosphate, acétate, lactate, alkyl(C₁-C₄)-sulfates, alkyl(C₁-C₄)-sulfonates ou alkyl(C₁-C₄)-aryl-sulfonates ;
c) les sels de diammonium ou triammonium quaternaire, en particulier ceux de formule (IV) suivante : 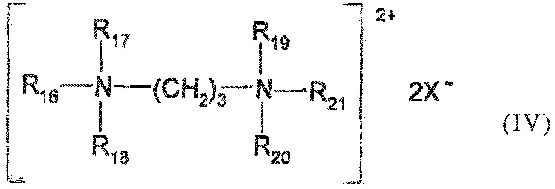 dans laquelle R₁₆ représente un groupe alkyle comportant à peu près de 16 à 30 atomes de carbone, en option hydroxylé et/ou interrompu par un ou plusieurs atome(s) d'oxygène, R₁₇ représente une entité choisie parmi un atome d'hydrogène, un groupe alkyle comportant de 1 à 4 atomes de carbone et un groupe de formule -(CH₂)₃-N⁺(R₁₆ₐ)(R₁₇ₐ)(R₁₈ₐ), R₁₆ₐ, R₁₇ₐ, R₁₈ₐ, R₁₈, R₁₉, R₂₀ et R₂₁ représentent des entités identiques ou différentes, choisies chacune parmi un atome d'hydrogène et un groupe alkyle comportant de 1 à 4 atomes de carbone, et X- représente un anion choisi dans l'ensemble constitué par les anions halogénures, acétate, phosphate, nitrate, alkyl(C₁-C₄)-sulfates, alkyl(C₁-C₄)-sulfonates et alkyl(C₁-C₄)-aryl-sulfonates, en particulier un anion méthyl-sulfate ou éthyl-sulfate ;
d) et les sels d'ammonium quaternaire comportant un ou plusieurs groupe(s) fonctionnel(s) de type ester, tels ceux de formule (V) suivante : 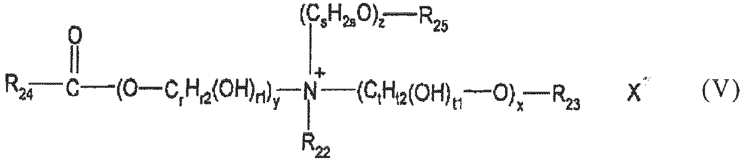 dans laquelle
R₂₂ représente une entité choisie parmi les groupes alkyle en C₁-C₆ et les groupes hydroxyalkyle ou dihydroxyalkyle en C₁-C₆,
R₂₃ représente une entité choisie parmi :
- les groupes de formule 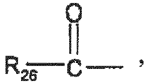
- les groupes hydrocarbonés en C₁-C₂₂, linéaires ou ramifiés, saturés ou insaturés, symbolisés par R₂₇,
- et un atome d'hydrogène,
R₂₅ représente une entité choisie parmi :
- les groupes de formule 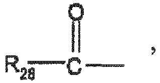
- les groupes hydrocarbonés en C₁-C₆, linéaires ou ramifiés, saturés ou insaturés, symbolisés par R₂₉,
- et un atome d'hydrogène,
R₂₄, R₂₆ et R₂₈ représentent des entités identiques ou différentes, choisies parmi les groupes hydrocarbonés en C₇-C₂₁, linéaires ou ramifiés, saturés ou insaturés,
les indices r, s et t, identiques ou différents, sont des nombres entiers valant de 2 à 6,
les indices r1 et t1, identiques ou différents, valent 0 ou 1,
et r2 + r1= 2 r et t1 + t2 = 2t,
l'indice y est un nombre entier valant de 1 à 10,
les indices x et z, identiques ou différents, sont des nombres entiers valant de 0 à 10,
et X- représente un anion simple ou complexe, organique ou inorganique,
sous réserve que la somme x + y + z vaille de 1 à 15, que si x vaut 0, alors R₂₃ représente R₂₇, et que si z vaut 0, alors R₂₅ représente R₂₉.

7. Composition conforme à l'une des revendications précédentes, **caractérisée en ce que** le ou les tensioactif(s) cationique(s) se trouve(nt) présent(s) en une proportion d'au moins 0,05 %, en poids rapporté au poids total de la composition.

8. Composition conforme à l'une des revendications précédentes, **caractérisée en ce que** le ou les tensioactif(s) non-ionique(s) est ou sont choisi(s) parmi les suivants :
- les alcools, alpha-diols et alkyl(C₁-C₂₀)-phénols polyéthoxylés, polypropoxylés et/ou polyglycérolés, comprenant au moins une chaîne grasse comportant de 8 à 18 atomes de carbone,
- les copolymères d'oxyde d'éthylène et d'oxyde de propylène, les esters d'acide gras et de sorbitane, en option éthoxylés, les esters d'acide gras et de saccharose, les esters d'acide gras polyalcoxylés, les alkyl-polyglycosides, en option alcoxylés, les esters d'alkylglucoside, les aldobionamides et les oxydes d'amine.

9. Composition conforme à l'une des revendications précédentes, **caractérisée en ce que** le ou les tensioactif(s) non-ionique(s) se trouve(nt) présent(s) en une proportion d'au moins 0,1 %, en poids rapporté au poids total de la composition.

10. Composition conforme à l'une des revendications précédentes, **caractérisée en ce qu'**elle contient un ou plusieurs tensioactif(s) cationique(s), en un rapport pondéral supérieur ou égal à 1 entre, d'une part, la quantité d'alcoxy-silane(s) à chaîne grasse, et d'autre part, la quantité de tensioactif(s) cationique(s).

11. Composition conforme à l'une des revendications précédentes, **caractérisée en ce qu'**elle contient un ou plusieurs tensioactif(s) non-ionique(s), en un rapport pondéral supérieur ou égal à 0,05 entre, d'une part, la quantité de tensio-actif(s) non-ionique(s), et d'autre part, la quantité d'alcoxy-silane(s) à chaîne grasse.

12. Composition conforme à l'une des revendications précédentes, **caractérisée en ce qu'**elle comprend aussi un ou plusieurs épaississant(s), présent(s) en une proportion de 0,01 à 20 %, en poids rapporté au poids total de la composition.

13. Composition conforme à l'une des revendications précédentes, **caractérisée en ce qu'**elle comprend aussi un ou plusieurs acide(s) organique(s), choisi(s) parmi l'acide acétique, l'acide propanoïque, l'acide butanoïque, l'acide lactique, l'acide malique, l'acide glycolique, l'acide ascorbique, l'acide maléique, l'acide phtalique, l'acide succinique, la taurine, l'acide tartrique, la glycine, l'acide glucuronique, l'acide gluconique et l'acide citrique,

14. Composition conforme à l'une des revendications précédentes, **caractérisée en ce qu'**elle comprend aussi un ou plusieurs additif(s) choisi(s) parmi les substances grasses non-siliceuses, les silicones, les particules solides, les agents réducteurs et les agents oxydants.

15. Composition conforme à l'une des revendications précédentes, **caractérisée en ce qu'**elle est aqueuse.

16. Composition conforme à l'une des revendications 1 à 14, **caractérisée en ce qu'**elle est anhydre.

17. Utilisation d'une composition conforme à l'une des revendications 1 à 16 pour le traitement de matières kératiniques, **caractérisée en ce qu'**elle consiste à appliquer sur ces matières, en une quantité efficace, une composition conforme à l'une des revendications 1 à 16, et ensuite, en option, à l'enlever par rinçage après un éventuel temps de pose, en présence ou non de chaleur.
